(19) Europäisches Patentamt
European Patent Office
Office européen des brevets

(11) Publication number: **0 182 499**
**B1**

(12) **EUROPEAN PATENT SPECIFICATION**

(45) Date of publication of patent specification: **12.04.89**

(21) Application number: **85307485.4**

(22) Date of filing: **17.10.85**

(51) Int. Cl.$^4$: **C 07 C 103/84,** C 07 C 102/00, C 07 C 153/067, A 61 K 31/195

(54) **N-(6-methoxy-5-(perfluoroalkyl)-1-naphtolyl)-N-metylglycines and their thionaphthoyl analogs.**

(30) Priority: **16.11.84 US 672015**

(43) Date of publication of application:
**28.05.86 Bulletin 86/22**

(45) Publication of the grant of the patent:
**12.04.89 Bulletin 89/15**

(84) Designated Contracting States:
**AT BE CH DE FR GB IT LI LU NL SE**

(56) References cited:
**US-A-4 439 617**

**Hansch and Leo, Substituent constants for correlation analysis in chemistry and biology, 48-49**

(73) Proprietor: **AYERST, MCKENNA AND HARRISON INC.**
**1025 Laurentian Boulevard**
**St. Laurent Quebec H4R 1J6 (CA)**

(72) Inventor: **Bellini, Francesco**
**3065 Graham Boulevard**
**Mount Royal Quebec H3R 1J8 (CA)**

Inventor: **Sestanj, Kazimir**
**2 Harper Road**
**Monmouth Junction New Jersey 08852 (US)**

(74) Representative: **Wileman, David Francis et al**
**c/o John Wyeth and Brother Limited**
**Huntercombe Lane South**
**Taplow Maidenhead Berkshire SL6 OPH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to N-[6-methoxy-5-(perfluoroalkyl)-1-naphthoyl]-N-methylglycine derivatives and their thionaphthoyl analogs, to processes for their preparation, to methods for using the derivatives, and to pharmaceutical preparations containing them. The derivatives have pharmaceutical properties which render them beneficial for the treatment of diabetes mellitus and associated conditions.

For many years diabetes mellitus has been treated with two established types of drugs, namely insulin and oral hypoglycemic agents. These drugs have benefited hundreds of thousands of diabetics by improving their well-being and prolonging their lives. However, the resulting longevity of diabetic patients has led to complications such as neuropathy, nephropathy, retinopathy, cataracts and atherosclerosis. These complications have been linked to the undesirable accumulation of sorbitol in diabetic tissue, which in turn result from the high levels of glucose characteristic of the diabetic patient.

In mammals, including humans, the key enzyme involved in the conversion of hexoses to polyols (e.g. the sorbitol pathway) is aldose reductase. J. H. Kinoshita and collaborators, see J. H. Kinoshita *et al.*, Biochem. Biophys. Acta, *158*,472 (1968) and references cited therein, have demonstrated that aldose reductase plays a central role in the etiology of galactosemic cataracts by effecting the conversion of galactose to dulcitol (galactitol) and that an agent capable of inhibiting aldose reductase can prevent the detrimental accumulation of dulcitol in the lens. Furthermore, a relationship between elevated levels of glucose and an undesirable accumulation of sorbitol has been demonstrated in the lens, peripheral nervous cord and kidney of diabetic animals, see A. Pirie and R. van Heyningen, Exp.Eye Res., *3*, 124 (1964); L. T. Chylack and J. H. Kinoshita, Invest. Ophthal., *8*,401 (1969) and J. D. Ward and R. W. R. Baker, Diabetol., *6*,531 (1970).

1,3-Dioxo-1H-benz[de]isoquinoline-2(3H)-acetic acid has been reported to be an effective inhibitor of aldose reductase, see D. Dvornik *et al.*, Science, *182*,1146 (1973), and to be useful for the treatment of diabetic complications such as diabetic cataracts, neuropathy, nephropathy and retinopathy, see K. Sestanj, N. Simard-Duquesne and D. Dvornik, U.S. Patent No. 3,821,383 June 28, 1974. Other compounds having a similar utility are the thioxo-1H-benz[de]isoquinoline-2(3H)-acetic acid derivatives of K. Sestanj, U.S. Patent 4,254,108, March 3, 1981 and 1H-benz[de]isoquinoline-2(3H)-acetic acid derivatives of K. Sestanj, U.S. Patent 4,254,109, March 3, 1981. Still other compounds having a similar utility are 2-thioxobenz[c,d]indole-1(2H)-acetic acid derivatives of K. Sestanj, U.S. Patent 4,369,188, January 18, 1983; N-naphthoylglycine derivatives of K. Sestanj *et al.*, U.S. Patent 4,439,617, March 27, 1984; N-(naphthalenylthioxomethyl)amino acid derivatives of K. Sestanj et al., U.S. Patent 4,391,816, July 5, 1983; N-[(2-naphthalenyl)thioxomethyl]glycine derivatives of K. Sestanj, U.S. Patent 4,447,452, May 8, 1984; and N-[[6-(lower alkoxy)-5-(trifluoromethylthio)-1-naphthalenyl]thioxomethyl]-N-(lower alkyl)-glycines of F. Bellini *et al.*, U.S. Patent 4,391,825, July 5, 1983. (S)-6-Fluoro-2,3-dihydrospiro(4H-1-benzopyran-4,4′-imidazolidine)-2′,5′-dione (sorbinil) is still another compound that has received attention because of its aldose reductase inhibiting properties (see M. J. Peterson *et al.*, Metabolism *28* (Suppl. 1), 456 (1979). Accordingly, these compounds represent an important new approach for the treatment of diabetes mellitus.

The present application discloses novel perfluoroalkyl-1-naphthoyl-N-methylglycines and their thionaphthoyl analogs, represented below by formula I, which are effective inhibitors of aldose reductase. These are structurally different from the above noted aldose reductase inhibitors.

The closest of the previously reported compounds is seen in U.S. Patent 4,439,617 (Example 52) and differs from the present derivatives by having different substituents, in that the compounds hereof have a perfluoroalkyl with 2—6 carbon atoms in the 5 position instead of the trifluoromethyl of the above patent.

The N-[6-methoxy-5-(perfluoroalkyl)-1-naphthoyl]-N-methylglycines and their thionaphthoyl analogs of this invention are represented by formula I

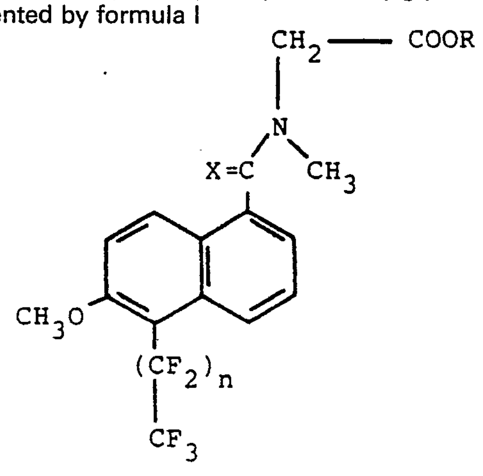

(I)

wherein n is an integer from 1 to 5, X is oxygen or sulphur and R is hydrogen or $C_{1-4}$ alkyl.

A preferred group of the compounds of the invention is represented by formula I wherein n is 2. Preferably R is hydrogen.

The N-[6-methoxy-5-(perfluoroalkyl)-1-naphthoyl]-N-methylglycine derivatives and their thionaphthoyl analogs can be prepared by methods analogous to those described in European Patent No. 59596.

A method is provided for preventing or relieving diabetes mellitus associated complications in a diabetic mammal by administering to said mammal a prophylactic or alleviating amount of the compound of formula I. Such complications include neuropathy, nephropathy, retinopathy and cataracts.

The compounds of formula I and therapeutically acceptable salts of those wherein R is hydrogen when admixed with a pharmaceutically acceptable carrier, form a pharmaceutical composition which can be used according to the preceding method.

The compounds of this invention, represented by formula I, can exist in rotameric forms. More explicitly, mesomerism imparts a partial double bond character to the carbon-nitrogen bond of the thioamide group. This partial double bond character leads to restricted rotation about the carbon nitrogen bond giving rise to cis and trans rotamers, the restricted rotation being augmented by the bulkiness of neighbouring groups. Interconversion of the rotamers is possible and is dependent on the physical environment. As evidenced by its physical properties, the thermodynamically more stable rotamer exists exclusively in the crystalline state of the compound and is the predominant isomer present in equilibrated solutions. Furthermore, the more stable rotamer is the more pharmacologically active. The less stable rotamer can be separated from the more stable rotamer by high performance liquid chromatography or by thin layer chromatography. The rotameric forms are included within the scope of this invention. For brevity, the compounds of this invention, including their rotameric forms, are referred to herein as compounds of formula I.

The term "alkyl" as used herein means a straight chain alkyl radical containing from one to four carbon atoms or a branched chain alkyl radical containing three of four carbon atoms and includes methyl, ethyl, propyl, 1-methylethyl, butyl, 1,1-dimethylethyl, and 1-ethyl-2-methylpropyl. Preferred alkyl radicals contain from one to three carbon atoms and most preferred is methyl.

The term "lower alkoxy" as used herein means a straight chain alkoxy radical containing one to four carbon atoms, preferably one to three carbon atoms, or a branched chain radical containing three or four carbon atoms, and includes methoxy, ethoxy, 1-methylethoxy, and butoxy.

The term "lower alkanoic acid" as used herein means both straight and branched chain alkanoic acids containing from two to four carbon atoms and includes acetic acid, propanoic acid and 2-methylpropionic acid.

The term "organic proton acceptor" as used herein means the organic bases or amines, for instance, triethylamine, pyridine, N-ethylmorpholine and 1,5-diazabicyclo[4.3.0]non-5-ene.

The N-[6-methoxy-5-(perfluoroalkyl)-1-naphthoyl]-N-methylglycines and their thionaphthoyl analogs of this invention may be administered to mammals, for example, man, cattle or rabbits, either alone or in dosage forms, i.e. capsules or tablets, combined with pharmacologically acceptable excipients, see below.

Advantageously the compounds of this invention may be given orally. However, the method of administering the present active ingredients of this invention is not to be construed as limited to a particular mode of administration. For example, the compounds may be administered topically directly to the eye in the form of drops of sterile, buffered ophthalmic solutions, preferably of pH 7.2—7.6. Also, they may be administered orally in solid form containing such excipients as starch, milk sugar and certain types of clay. They may also be administered orally in the form of solutions or they may be injected parenterally. For parenteral administration they may be used in the form of a sterile solution, preferably of pH 7.2—7.6, containing a pharmaceutically acceptable buffer.

The dosage of the N-[6-methoxy-5-(perfluoroalkyl)-1-naphthoyl]-N-methylglycines and their thionaphthoyl analogs will vary with the form of administration and the particular compound chosen. Furthermore, it will vary with the particular host under treatment. Generally, treatment is initiated with small dosages substantially less than the optimal dose of the compound. Thereafter, the dosage is increased by small increments until efficacy is obtained. In general, the compounds of this invention are most desirably administered at a concentration level that will generally afford effective results without causing harmful or deleterious side effects. For topical administration, a 0.05—0.2% solution may be administered dropwise to the eye. The frequency of instillation varies with the subject under treatment from a drop every two or three days to once daily. For oral or parenteral administration a preferred level of dosage ranges from about 0.1 mg to about 200 mg per kilo of body weight per day, although aforementioned variations will occur. However, a dosage level that is in the range of from about 3.0 mg to about 30 mg per kilo of body weight per day is most satisfactory.

Unit dosage forms such as capsules, tablets and pills may contain from about 5.0 mg to about 250 mg of the active ingredients of this invention with a pharmaceutical carrier. Thus, for oral administration, capsules can contain from between about 5.0 mg to about 250 mg of the active ingredients of this invention with or without a pharmaceutical diluent. Tablets, either effervescent or noneffervescent, can contain between about 5.0 to 250 mg of the active ingredients of this invention together with conventional pharmaceutical carriers. Thus, tablets which may be coated and either effervescent or noneffervescent, may be prepared according to the known art. Inert diluents or carriers, for example, magnesium carbonate or lactose, can be used together with conventional disintegrating agents for example, magnesium stearate.

The N-[6-methoxy-5-(perfluoroalkyl)-1-naphthoyl]-N-methylglycines and their thionaphthoyl analogs also can be used in combination with insulin or oral hypoglycemic agents to produce a beneficial effect in

the treatment of diabetes mellitus. In this instance, commercially available insulin preparations or oral hypoglycemic agents, exemplified by acetohexamide, chlorpropamide, tolazamide, tolbutamide and phenformin, are suitable. The compounds hereof can be administered sequentially or simultaneously with insulin or the oral hypoglycemic agent. Suitable methods of administration, compositions and doses of the insulin preparation or oral hypoglycemic agent are described in medical textbooks; for instance, "Physicians' Desk Reference", 36 ed., Medical Economics Co., Oradell, N.J., U.S.A., 1982. When used in combination, the N-[6-methoxy-5-(perfluoroalkyl)-1-naphthoyl]-N-methylglycines and their thionaphthoyl analogs are administered as described previously. The N-[6-methoxy-5-(perfluoroalkyl)-1-naphthoyl]-N-methylglycines and their thionaphthoyl analogs can be administered with the oral hypoglycemic agent in the form of a pharmaceutical composition comprising effective amounts of each agent.

The aldose reductase inhibiting property of the compounds of this invention and the utilization of the compounds in preventing, diminishing and alleviating diabetic complications are demonstrable in experiments using galactosemic rats, see Dvornik *et al.*, cited above. Such experiments are exemplified hereinbelow after the listing of the following general comments pertaining to these experiments:

(a) Four or more groups of six male rats, 50—70 g, Sprague-Dawley strain, were used. The first group, the control group, was fed a mixture of rodent laboratory chow, (Purina) and glucose at 20% (w/w%) concentration. The untreated galactosemic group was fed a similar diet in which galactose is substituted for glucose. The third group was fed a diet prepared by mixing a given amount of the test compound with the galactose containing diet. The concentration of galactose in the diet of the treated groups was the same as that for the untreated galactosemic group.

(b) After four days, the animals were killed by decapitation. The eyeballs were removed and punctured with a razor blade; the freed lenses were rolled gently on filter paper and weighed. The sciatic nerves were dissected as completely as possible and weighed. Both tissues were frozen and can be kept up to two weeks before being analyzed for dulcitol.

(c) The polyol determination was performed by a modification of the procedure of M. Kraml and L. Cosyns, Clin. Biochem., *2*,373 (1969). Only two minor reagent changes were made: (a) The rinsing mixture was an aqueous 5% (w/w) trichloroacetic acid solution and (b) the stock solution was prepared by dissolving 25 mg of dulcitol in 100 ml of an aqueous trichloroacetic acid solution. [N.B.: For each experiment the average value found in the tissue from rats fed the glucose diet was subtracted from the individual values found in the corresponding rat tissue to obtain the amount of polyol accumulated.]

The following tabulated results show that the N-[6-methoxy-5-(perfluoroalkyl)-1-naphthoyl]-N-methylglycines and their thionaphthoyl analogues of this invention diminish the accumulation of dulcitol in the lenses and sciatic nerves of rats fed galactose. The figures under L, N and D represent the percentage decrease of dulcitol accumulation in the tissues of the lens, sciatic nerve and diaphragm, respectively, for treated rats as compared with untreated rats.

Examination of the results tabulated below shows that the perfluoroalkyls of this invention are surprisingly well suited as aldose reductase inhibitors. For example, compound No. 4, N-[[5-(heptafluoropropyl)-6-methoxy-1-naphthalenyl]carbonyl]-N-methylglycine at a dose of 10 mg/kg/day gives comparable results to compound No. 7 at 11 mg/kg/day. The latter compound, N-[[5-(trifluoromethyl)-6-methoxy-1-naphthalenyl]-thioxomethyl]-N-methylglycine, which is also known as tolrestat, is presently undergoing clinical trials. Compound 4 as contrasted to tolrestat does not have sulphur in the molecule. This provides an opportunity to have a non-sulphur containing aldose reductase inhibitor. In a similar manner compound No. 3. N-[[5-(heptafluoropropyl)-6-methoxy-1-naphthalenyl]thioxomethyl]-N-methylglycine, at 5 mg/kg/day, is superior to tolrestat at 4 mg/kg/day and approaches the activity of tolrestat at 11 mg/kg/day. Thus, compound No. 3 is about twice as active as tolrestat.

| Test compound | n | X | % inhibition in vitro $10^{-7}$M | mg/kg day | % lowering dulcitol accumulation in vivo L | N | D |
|---|---|---|---|---|---|---|---|
| N-[[5-(Pentafluoroethyl)-6-methoxy-1-naphthalenyl]-thioxomethyl]-N-methylglycine | 1 | S | 71 | 24.5 | 29 | 93 | 93 |
| N-[[5-(Pentafluoroethyl)-6-methoxy-1-naphthalenyl]-carbonyl]-N-methylglycine | 1 | O | 56 | 29 | 0 | 56 | 68 |
| N-[[5-(Heptafluoropropyl)-6-methoxy-1-naphthalenyl]thioxomethyl]-N-methylglycine | 2 | S | 75 | 5 | 20 | 52 | 76 |
| N-[[5-(Heptafluoropropyl)-6-methoxy-1-naphthalenyl]-carbonyl]-N-methylglycine | 2 | O | 63 | 10 | 19 | 45 | 38 |
| N-[[5-(Tridecafluorohexyl)-6-methoxy-1-naphthalenyl]-thioxomethyl]-N-methylglycine | 5 | S | 40 | 26 | 0 | 22 | 51 |
| N-[[5-(Tridecafluorohexyl)-6-methoxy-1-naphthalenyl]-carbonyl]-N-methylglycine | 5 | O | 47 | 25 | 0 | 0 | 0 |
| N-[[5-(Trifluoromethyl)-6-methoxy-1-naphthalenyl]-thioxomethyl]-N-methyl glycine | 0 | S | 79 | 4<br>11 | 0<br>14 | 35<br>86 | 80<br>89 |
| N-[[5-(Trifluoromethyl)-6-methoxy-1-naphthalenyl]-carbonyl]-N-methylglycine | 0 | O | 57 | 10 | 0 | 25 | 20 |

This invention also provides a process for preparing a compound of formula I as defined hereinabove or a therapeutically acceptable salt with an organic or inorganic base of the compound of formula I wherein R is hydrogen; said processes comprising one of the following:

(i) hydrolysing a compound of formula IX

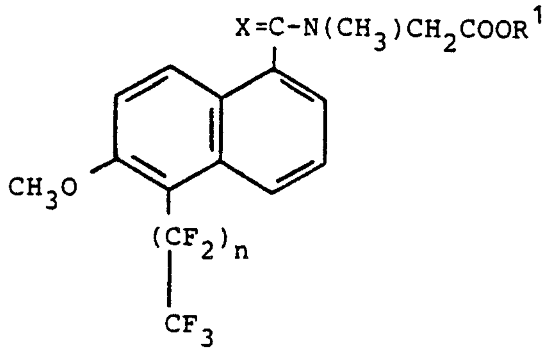

IX

where $COOR^1$ is an ester function, and n and X are as defined above to give a compound of formula I wherein R is hydrogen;

(ii) reacting a compound of formula Va

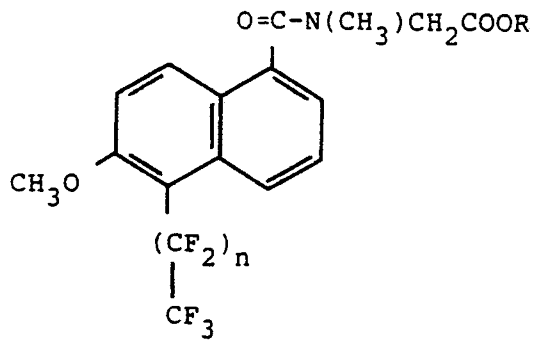

Va

where R and n are as defined above with phosphorous pentasulphide to give a compound of formula I wherein X is sulphur;

(iii) coupling a compound of formula

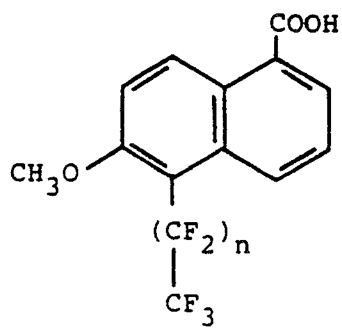

IV

wherein n is as defined above with a compound of formula

$$HN(CH_3)CH_2COO(C_{1-4}\ alkyl) \qquad (X)$$

to give a compound of formula I wherein R is lower alkyl;

(iv) reacting a compound of formula

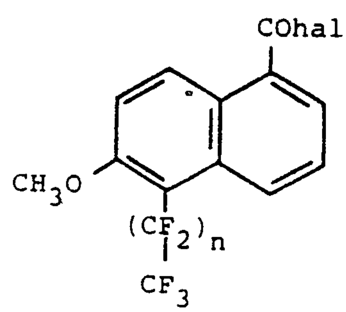

(XI)

in which formula n is as defined above and hal represents chlorine or bromine, with N-methylglycine to give a compound of formula I wherein X is oxygen and R is hydrogen;

(v) perfluoroalkylating a compound of formula

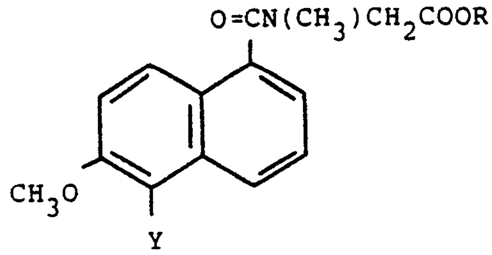

(XII)

wherein Y represents chlorine, bromine or iodine and R is as defined above with an alkylating agent containing the group $CF_3(CF_2)_n$— to give a compound of formula I wherein X = oxygen or

(vi) reacting a compound of formula I wherein R is H with an organic or inorganic base to form a therapeutically acceptable salt or

(vii) acidifying a salt of a compound of formula I to give a compound of formula I wherein R is hydrogen.

With reference to process (i) above the ester of formula IX may be hydrolysed with a hydrolysing agent to give the corresponding product of formula I in which R is hydrogen. Generally speaking, this conversion is most conveniently performed by employing a base as the hydrolysing agent. The hydrolysis may be performed in the presence of sufficient water, followed by acidification of the reaction mixture, to yield the desired acid. However, it should be understood that the manner of hydrolysis for the process of this invention is not intended to be limited to basic hydrolysis since hydrolysis under acidic conditions and other variations, for example, treatment with lithium iodide in collidine (see L. F. Fieser and M. Fieser, "Reagents for Organic Synthesis", John Wiley and Sons, Inc., New York, 1969, pp 615—617), also are applicable. Hydrolysis under acidic conditions is preferred when the ester is a tert butyl ester.

For basic hydrolysis, a preferred embodiment involves subjecting the ester to the action of a strong base, for example, sodium or potassium hydroxide, in the presence of sufficient water to effect hydrolysis of the ester. The hydrolysis may be performed using a suitable solvent, for example, methanol, ethanol or 2-methoxyethanol. The reaction mixture may be maintained at a temperature of from about 25°C to 100°C or at the reflux temperature of the solvent employed until hydrolysis occurs. Usually ten minutes to six hours is sufficient for this hydrolysis. The reaction mixture is then rendered acidic with an acid, for example, acetic acid, hydrochloric acid or sulphuric acid to release the free acid.

With reference to process (ii) above amidoester of formula (Va) ($R = C_{1-4}$ alkyl) may be reacted, for example, under anhydrous conditions with about two to five molar equivalents of phosphorus pentasulfide in an inert solvent, e.g. xylene or toluene, to obtain the corresponding thioxoester of formula I. This reaction is performed conveniently at temperature ranging from 80 to about 150°C and at times ranging from 20 minutes to four hours. Preferably, the reaction is performed in the presence of an organic base for instance, N-ethyl morpholine, triethylamine or pyridine. In a similar manner acids of formula Va (R = H) are reacted with $P_2S_5$ to give corresponding acids of formula I. It should be noted that the standard first step of the work up of the pentasulfide reaction mixture requires that the reaction mixture be decomposed in water. This action causes any corresponding thioacid, present in the reaction mixture as a result of the carboxy group reacting with phosphorus pentasulfide, to be converted to the desired carboxylic acid.

With reference to process (iii) the coupling of the naphthalenecarboxylic acid IV and the amino ester X is done preferably by the "carboxyl activation" coupling procedure. Descriptions of carboxyl-activating groups are found in general textbooks of peptide chemistry; for example, K. D. Kopple, "Peptides and Amino Acids", W. A. Benjamin, Inc., New York, 1966, pp 45—51, and E. Schroder and K. Lubke, "The Peptides"; Vol. 1, Academic Press, New York, 1965, pp. 77—128. Examples of the activated form of the terminal carboxyl are the acid chloride, acid bromide, anhydride, azide, activated ester, or O-acyl urea obtained from a dialkylcarbodiimide e.g. dicyclohexylcarbodiimide (DCC). Preferred activated forms of the carboxyl are the acid chloride or the 1-benzotriazolyl, 2,4,5-trichlorophenyl or succinimido activated esters.

With reference to process (iv) the amidoacid of formula I wherein R is oxygen also can be prepared by a previously reported process involving the reaction of the appropriate naphthalenecarboxylic acid halide with N-methylglycine in the presence of a base (proton acceptor). This process has been used to prepare N-[(1-naphthalenyl)carbonyl]glycine, see Chem.Abstr., 61, 4333 f (1964) for E. Cioranescu, et al., Rev. Chim., Acad., Rep. Populaire Roumaine, 7(2), 755 (1962). However, this process for preparing N-[(1-naphthalenyl)carbonyl]glycine is generally inferior, based on yields, to the described process above.

With reference to process (v) methods for perfluoroalkylating the compound of formula (XII) by displacing Y are well known in the art. For example an Ullmann reaction may be carried out using a perfluoroalkylating agent of formula $CF_3(CF_2)_nY^1$ wherein $Y^1$ is bromine or iodine. The Ullman reaction is extensively described in the literature — see for example — P. E. Fanta Chemical Reviews, *38*, 139 (1946); *64* 613 (1964) and Synthesis 1974, 9. Such a reaction is conveniently carried out in the presence of an inert solvent such as pyridine and using finely divided copper. Preferably Y and $Y^1$ are both iodine.

The salts of the acids of formula I included within the scope of this invention are formed by process vi).

The acid is transformed in excellent yield into the corresponding therapeutically acceptable salt by neutralization of said acid with the appropriate inorganic or organic base. The salts are administered usually in the same manner as the parent acid compounds. Suitable inorganic bases to form these salts

include, for example, the hydroxides, carbonates or bicarbonates of the therapeutically acceptable alkali metals or alkaline earth metals, for example, sodium, potassium, magnesium and calcium. Suitable organic bases include the following amines: benzylamine; lower mono-, di and trialkylamines, the alkyl radicals of which contain up to three carbon atoms, such as methylamine, dimethylamine, triethylamine, ethylamine, di and triethylamine and methylethylamine; mono- di and trialkanolamines, the alkanol radicals of which contain up to three carbon atoms, for example, mono-, di and triethanolamine; alkylenediamines which contain up to six carbon atoms, such as hexamethylenediamine; cyclic saturated or unsaturated bases containing up to six carbon atoms, such as pyrrolidine, piperidine, morpholine, piperazine and their N-alkyl and N-hydroxyalkyl derivatives, such as N-methyl-morpholine and N-(2-hydroxyethyl)-piperidine, as well as pyridine. Furthermore, there may be mentioned the corresponding quaternary salts, such as the tetraalkyl (for example tetramethyl), alkyl-alkanol (for example methyl-triethanol and trimethyl-monoethanol) and cyclic ammonium salts, for example the N-methylpyridinium, N-methyl-N-(2-hydroxyethyl)morpholinium, N,N-dimethylmorpholinium, N-methyl-N-(2-hydroxyethyl)-morpholinium, N,N-dimethylpiperidinium salts, which are characterised by having good water-solubility. In principle, however, there can be used all the ammonium salts which are physiologically compatible.

The transformations to the salts can be carried out by a variety of methods known in the art. For example, in the case of the inorganic salts, it is preferred to dissolve the acid of formula I in water containing at least one equivalent amount of a hydroxide, carbonate, or bicarbonate corresponding to the inorganic salt desired. Advantageously, the reaction is performed in a water-miscible, inert organic solvent, for example, methanol, ethanol and dioxane, in the presence of water. For example, such use of sodium hydroxide, sodium carbonate or sodium bicarbonate gives a solution of the sodium salt. Evaporation of the solution or addition of a water-miscible solvent of a more moderate polarity, for example, a lower alkanol, for instance, butanol, or a lower alkanone, for instance, ethyl methyl ketone, gives the solid inorganic salt if that form is desired.

To produce an amine salt, the acidic compound of formula I may be dissolved in a suitable solvent of either moderate or lower polarity, for example, ethanol, methanol, ethyl acetate, diethyl ether and benzene. At least an equivalent amount of the amine corresponding to the desired cation is then added to that solution. If the resulting salt does not precipitate, it can usually be obtained in solid form by addition of a miscible diluent of lower polarity, for example, benzene or petroleum ether, or by evaporation. If the amine is relatively volatile, any excess can easily be removed by evaporation. It is preferred to use substantially equivalent amounts of the less volatile amines.

Salts wherein the cation is quaternary ammonium may be produced by mixing the acid of formula I with an equivalent amount of the corresponding quaternary ammonium hydroxide in water solution, followed by evaporation of the water.

Treatment of a salt of a compound of formula I with an acid, e.g. as mineral acid such as hydrochloric acid gives the free acid of formula I.

In a preferred process the N-[6-methoxy-5-(perfluoroalkyl)-1-naphthoyl]-N-methylglycines and their thionaphthoyl analogues are prepared by the following reaction scheme wherein $COOR^1$ represents an ester group, particularly COOR where R is $C_{1-4}$ alkyl:

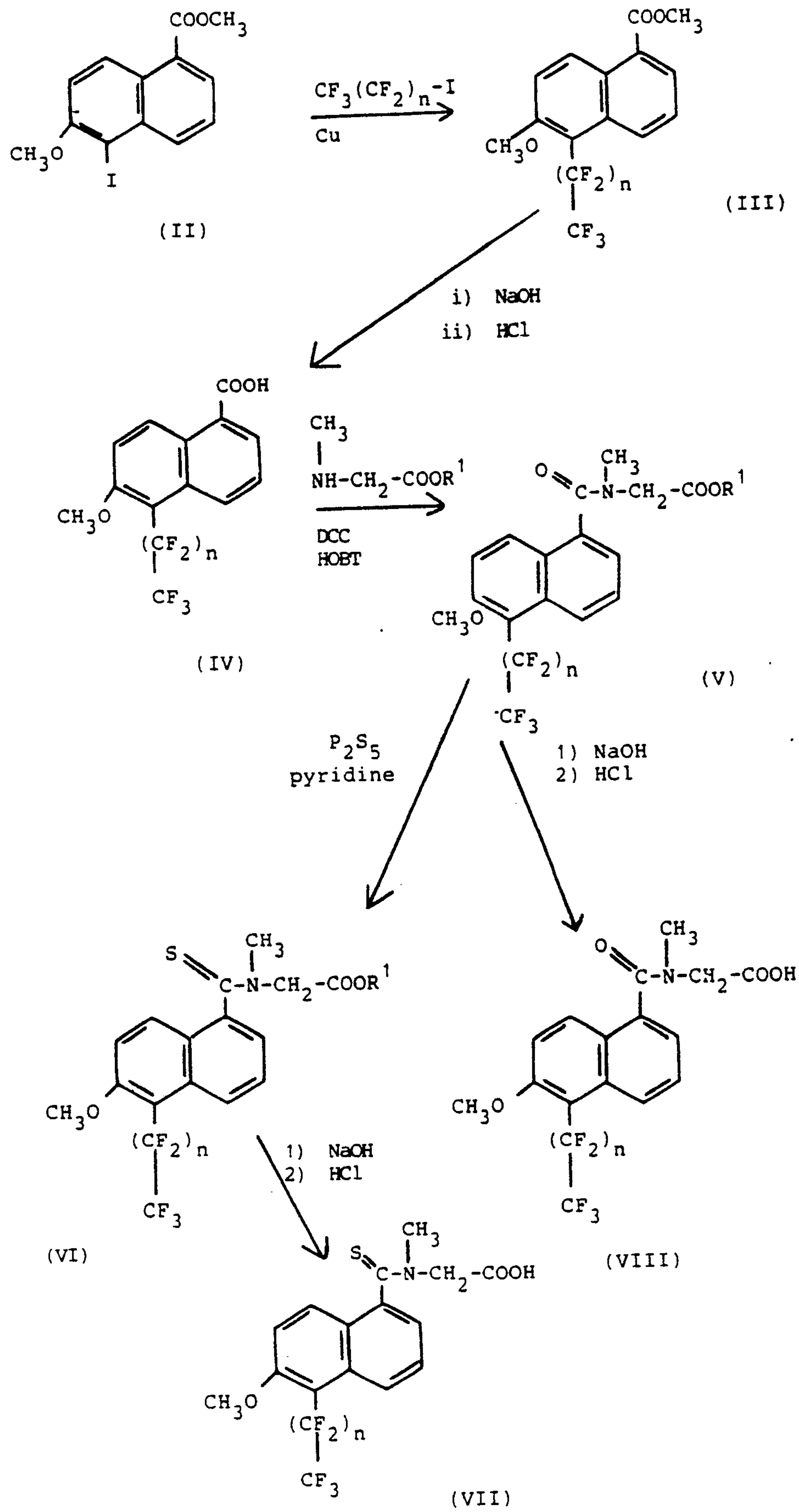


(II)

(III)

(IV)

(V)

(VI)

(VIII)

(VII)

In a preferred embodiment of this process perfluoroalkylating 6-methoxy-5-iodo-1-naphthalene carboxylic acid, methyl ester (II), a known compound, forms the corresponding perfluoroalkyl compound, (III), hydrolyzing said perfluoroalkyl compound to the corresponding carboxylic acid, (IV), coupling said acid with sarcosine methyl ester forms the corresponding substituted N-methylglycine, methyl ester, (V), $R^1$ = Me, hydrolyzing said substituted N-methylglycine, methyl ester forms the corresponding substituted N-methylglycine, (VIII). The N-methylglycine, methyl ester, (V), $R^1$ = Me, can also be reacted with $P_2S_5$ to form the corresponding substituted thioxomethyl-N-methylglycine, methyl ester, (VI), and hydrolyzing the latter forms the corresponding substituted thioxomethyl-N-methylglycine (VII).

The following examples illustrate the invention:

Example 1

N[[6-Methoxy-5-(pentafluoroethyl)-1-naphthalenyl]carbonyl]-N-methylglycine (VIII, n = 1)

a) 6-Methoxy-5-iodo-1-naphthalene carboxylic acid, methyl ester (5 g, 0.0146 mol, Example 1F of U.S. Patent 4,439,627), 1-iodo-pentafluoro-ethane (10.8 g, 0.0438 mol) activated copper powder (3 g), in a manner similar to Y. Kobayashi et al., J.Chem. Soc. Perkin I, 2755 (1980) and dry pyridine (45 ml) were heated in a pressure bottle to 120°C for 20 hours. The mixture was cooled to room temperature, the precipitate filtered off, washed on the filter with ethyl acetate, and the filtrate washed with 2N hydrochloric acid, water, sodium bicarbonate, water and dried over anhydrous magnesium sulfate. The solvent was evaporated off, water and ethanol were added to the residue and the mixture left in a refrigerator. The product, 6-methoxy-5-(pentafluoroethyl)-1-naphthalenecarboxylic acid, methyl ester, was separated by filtration, washed with cold ethanol and dried (2.8 g). An additional amount (2 g) was obtained from the mother liquor, nmr ($CDCl_3$): δ 3.95 (s, 6H, $OCH_3$); δ 8.1 (m, 5H, $H_{ar}$) ms: m/e 334 ($M^+$), 303, 265, 234.

b) The 6-methoxy-5-(pentafluoroethyl)-1-naphthalenecarboxylic acid, methyl ester (2.82 g, 8.44 mmol), methanol (50 ml) and 2N aqueous sodium hydroxide (8.43 ml) were stirred at room temperature overnight. The mixture was cooled to 0°C, neutralized with 1N aqueous hydrochloric acid to pH 8 and the methanol evaporated off. Water was added to the residue and neutral impurities were removed by extraction with ethyl acetate. The aqueous layer was cooled to 0°, acidified to pH 1—3 with 1N aqueous hydrochloric acid, the product extracted with ethylacetate, the extract washed with brine and dried over anhydrous $MgSO_4$. Evaporation gave 2.14 g of crude 6-methoxy-5-(pentafluoroethyl)-1-naphthalene carboxylic acid which was used, without further purification, in the next step. nmr (DMSO): δ 4.05 (s, 3H, $OCH_3$); δ 8.0 (m, 4H, $H_{ar}$); δ 9.1 (d, 1H, J=9, $H_{ar}$) ms: m/e 320 ($M^+$), 303, 251, 203.

c) The crude 6-methoxy-5-(pentafluoroethyl)-1-naphthalene carboxylic acid (3.65 g, 11.4 mmol), 1-hydroxybenzotriazole (2.31 g, 17.1 mmol), dry distilled dimethylformamide (35 ml) and dicyclohexylcarbodiimide (2.82 g, 13.7 mmol) were stirred at room temperature for 1 hour. The mixture was cooled to 0°C, a solution of sarcosine methyl ester hydrochloride (3.18 g, 22.8 mmol) in dimethylformamide (30 ml) and N-ethyl morpholine (2.92 ml, 22.8 mmol) was added and stirring continued for 2 hours at room temperature. After cooling to 0°C, the precipitate (dicyclohexyl urea) was removed by filtration, the filtrate evaporated and the residue purified by HPLC (ethyl acetate/hexane 1:1, Waters Prep LC 500A). 1.5 g of pure, and 2.5 g of less pure N-[[6-methoxy-5-(pentafluoroethyl)-1-naphthalenyl]carbonyl]-N-methylglycine, methyl ester were obtained. nmr (DMSO): δ 2.75 and 3.25 (2s, 3H, N—$CH_3$, rotamers); δ 3.75 (s, 3H, $OCH_3$); δ 3.97 (s, 3H, $OCH_3$); δ 4.35 (broad, 2H, N—$CH_2$—CO); δ 7.7 (m, 5H, $H_{ar}$). ms: m/e 405 ($M^+$), 303, 195, 102.

d) To a solution of N-[[6-methoxy-5-(pentafluoroethyl)-1-naphthalenyl]carbonyl]-N-methylglycine, methyl ester (1.5 g, 3.7 mmol) in 2-methoxy-ethanol (30 ml) was added 2N aqueous sodium hydroxide (3.7 ml) at 0°C, under nitrogen atmosphere. Stirring was continued for 3 hours at room temperature. The reaction mixture was cooled to 0°C, neutralized (pH 8) with 1N aqueous hydrochloric acid, the solvent evaporated, the residue triturated with water, neutral material extracted with ethylacetate and the aqueous layer acidified to pH 3 with 1N aqueous hydrochloric acid and the product extracted with ethyl acetate. The extract was washed with water, dried over anhydrous magnesium sulfate, the solvent evaporated off and the residue crystallized from ethyl acetate-hexane. Yield: 700 mg of the pure N-[[6-methoxy-5-(pentafluoroethyl)-1-naphthalenyl]carbonyl]-N-methylglycine. An additional 170 mg were obtained from the mother liquor. Anal Calcd: C, 52.18% H, 3.61%, N, 3.58% Found: C, 52.31% H, 3.67% N, 3.58%. ir (nujol): 2500, 1900, 1725, 1580 $cm^{-1}$. uv, λmax (ε): 336 (3300), 324 (3000), 297 (5700), 285 (6200), 227 (53,400). nmr (DMSO): δ 2.75 and 3.1 (2s, 3H, N—$CH_3$, rotamers); δ 3.85 and 4.2 (m, 2H, $CH_2$); δ 4.0 (s, 3H, O—$CH_3$); δ 7.7 (m, 5H, $H_{ar}$). ms: m/e 391 ($M^+$), 346, 303.

Example 2

N-[[6-methoxy-5-(pentafluoroethyl)-1-naphthalenyl]thioxomethyl]-N-methylglycine (VI, n = 1)

a) Crude N-[[6-methoxy-5-(pentafluoroethyl)-1-naphthalenyl]carbonyl]-N-methylglycine, methyl ester, prepared as in Example 1 from 5.3 g or 13.4 mmol of 6-methoxy-5-(pentafluoroethyl)-1-naphthalene carboxylic acid, used without chromatrographic purification, was refluxed and stirred in dry pyridine (100 ml) with phosphorus pentasulfide (6 g) for 4 hours. After stirring overnight at room temperature, the mixture was hydrolyzed by addition of warm water, extracted with ethyl acetate, and the extract washed with 3N aqueous hydrochloric acid, water, sodium bicarbonate and brine. After drying and evaporation, the impure residue was dissolved in 25% ethyl acetate in hexane and the solution was filtered through silica gel. Evaporation of the filtrate and drying afforded 2.3 g of fairly pure N-[[6-methoxy-5-(pentafluoroethyl)-1-naphthalenyl]thioxomethyl]-N-methylglycine, methyl ester. NMR: ($CDCl_3$): Compatible (mixture of rotamers). ms: m/e 421 ($M^+$), 406, 388, 362, 319, 303.

b) The N-[[6-methoxy-5-(pentafluoroethyl)-1-naphthalenyl]thioxomethyl]-N-methylglycine, methyl ester (2.3 g, 5.46 mmol), 2-methoxyethanol (50 ml) and 2N aqueous sodium hydroxide (5.46 ml) were mixed at 0°C and the mixture stirred at room temperature for 3 hours (until disappearance of the ester spot in t.l.c. with ethyl acetate/hexane 1:1). The mixture was cooled to 0°C, neutralized to pH 8 with 1N aqueous hydrochloric acid, evaporated, the residue triturated with water, the neutral material extracted with ethyl acetate and the aqueous layer acidified with 1N aqueous hydrochloric acid to pH 3. The product was extracted with ethyl acetate, the extract washed with water, dried over anhydrous $MgSO_4$ and evaporated. The residue was purified by crystallization from chloroform/hexane to obtain 700 mg of the first crop and an additional 568 mg of the crude material from the mother liquor. Recrystallization of the first crop afforded 400 mg of the pure N-[[6-methoxy-5-(pentafluoroethyl)-1-naphthalenyl]thioxomethyl]-N-methylglycine. The recrystallization of mother liquor gave further 291 mg. mp. 164—165°C. Anal. Calcd: C, 50.13% H, 3.46% N, 3.44% Found: C, 49.42%, H, 3.58% N, 3.40%. nmr (DMSO): δ 2.95 (s, 3H, N—$CH_3$); δ 3.97 (s, 3H, $OCH_3$); δ 4.9 (q, 2H, $CH_2$); δ 7.1—8.4 (m, 5H, $H_{ar}$). ir (nujol): 1700, 1723, 1750 $cm^{-1}$ uv, λmax (ε): 338 (3990), 269 (13,000), 227 (45,400) ms: m/e 407 ($M^+$), 363, 319.

## Example 3

N-[[5-(Heptafluoropropyl)-6-methoxy-1-naphthalenyl]carbonyl]-N-methylglycine (VIII, n = 2)

a) The 6-Methoxy-5-iodo-1-naphthalene carboxylic acid, methyl ester (5 g, 0.0146 mol), 1-iodo-heptafluoro-propane (8.9 g, 4.35 ml, 0.03 mol), activated copper powder (2.8 g, 0.044 at) and dry distilled dimethylformamide (35 ml) were heated with stirring for 4 hours at 150° in a pressure bottle. The reaction mixture was cooled to room temperature, the solid removed by filtration and washed on the filter with ether. The filtrate was washed with water and dried over anhydrous magnesium sulfate. The solvent was evaporated and the residue left in a refrigerator to crystallize (5.6 g). A small amount of 5-(heptafluoropropyl)-6-methoxy-1-naphthalene carboxylic acid, methyl ester was recrystallized from ethanol. m.p. 74—75°C. ms: m/e 384 ($M^+$), 353, 265. nmr ($CDCl_3$): δ 4.0 (s, 6H, $OCH_3$); δ 8.25 (m, 5H, $H_{ar}$).

b) The 5-(heptafluoropropyl)-6-methoxy-1-naphthalene carboxylic acid, methyl ester (5.6 g, 0.0146 mol), methanol (40 ml) and 4N aqueous sodium hydroxide (7.3 ml) were stirred at room temperature for 30 minutes and then refluxed for 4 hours. After standing overnight, the mixture was cooled to 0° and neutralized with aqueous 1N hydrochloric acid. Methanol was evaporated, the residue triturated with water and the mixture acidified (pH ~ 3) with 1N aqueous hydrochloric acid. The pasty precipitate was extracted with ethyl acetate, the organic layer washed with water, dried over anhydrous magnesium sulfate and evaporated. The crude product, 5-(heptafluoropropyl)-6-methoxy-1-naphthalene carboxylic acid, (5.15 g) was used in the next step without further purification. One part of the product was recrystallized from chloroform-hexane for spectral identification. ms: m/e 370, 251, 203. nmr ($CDCl_3$): δ 4.0 (s, 3H, $OCH_3$); δ 7.4—9.35 (m, 5H, $H_{ar}$); δ 10.7 (b, 1H, COOH).

c) The crude acid (5.15 g, 0.0139 mol), 1-hydroxybenzotriazole (2.82 g, 0.021 mol), dry distilled dimethylformamide (40 ml) and dicyclohexylcarbodiimide (3.44 g, 0.0167 mol) were stirred for 1 hour at room temperature. A solution of sarcosine methyl ester hydrochloride (3.88 g, 0.0278 mol) in dimethylformamide (30 ml) was added to the above mixture followed by N-ethyl morpholine (3.55 ml). The reaction mixture was stirred overnight, the precipitate (dicyclohexyl urea) removed by filtration, the filtrate evaporated to dryness and the residue dissolved in ethyl acetate. The solution was washed with sodium bicarbonate and water, dried over anhydrous magnesium sulfate and evaporated. The residue was purified by chromatography using Waters Prep LC 500A instrument with ethyl acetate-hexane (1:1) system. Yield 3.3 g (a very pure fraction amounted to 1.5 g) N-[[5-(heptafluoropropyl)-6-methoxy-1-naphthalenyl]-carbonyl]-N-methylglycine methyl ester. ms: m/e 455, 353, 102. nmr ($CDCl_3$): δ 2.85 (s, 3H, N—$CH_3$); δ 3.4 (q, 2H, $CH_2$); δ 3.82 (s, 3H, $OCH_3$); δ 3.95 (s, 3H, $OCH_3$); δ 7.2—8.4 (m, 5H, $H_{ar}$).

d) To a solution of the N-[[5-(heptafluoropropyl)-6-methoxy-1-naphthalenyl]carbonyl]-N-methylglycine, methyl ester (1.52 g, 3.34 mmol) in 2-methoxy-ethanol (20 ml) was added 2N aqueous sodium hydroxide (3.34 ml) at 0°C under nitrogen atmosphere. Stirring was continued at room temperature for 2 hours. The mixture was cooled to 0°C, neutralized with 1N aqueous hydrochloric acid to pH 7, the solvent evaporated, the residue dissolved in water and acidified to pH 3. The product was isolated by filtration, washed with water and crystallized from chloroform-hexane giving 1.14 g of N-[[5-(heptafluoropropyl)-6-methoxy-1-naphthalenyl]carbonyl]-N-methylglycine. m.p. 195—197°C. Anal. Calcd: C, 49.02% H, 3.20% N, 3.18%. Found: C, 48.29% H, 3.20% N, 3.11%. ir (nujol): 2500, 1900, 1725, 1580, 1220, 1120 $cm^{-1}$. uv, λmax (ε): 336 (3400), 324 (3120), 298 (5960), 286 (6310), 227 (52,600). nmr (DMSO): δ 2.75 (s, 3H, N—$CH_3$); δ 3.98 (s, 3H, O—$CH_3$); δ 4.1 (m, 2H, N—$CH_2$); δ 7.75 (m, 5H, $H_{ar}$). ms: m/e 441 ($M^+$), 396, 353, 322, 69 also [442 $(M + 1)^+$, 443 $(M + 2)^+$.

## Example 4

N-[[5-heptafluoropropyl)-6-methoxy-1-naphthalenyl]thioxomethyl]-N-methylglycine (VI, n = 2)

a) N-[[5-(Heptafluoropropyl)-6-methoxy-1-naphthalenyl]carbonyl]-N-methylglycine methyl ester (1.78 g, 0.0039 mol), prepared as in Example 3, dry pyridine (30 ml) and phosphorus pentasulfide (2 g, 0.009 mol) were refluxed under nitrogen for 6 hours. The still warm mixture was poured into warm water,

extracted with ethyl acetate, the extract washed with 3N aqueous hydrochloric acid, saturated sodium bicarbonate and brine. The solvent was evaporated, the oily residue was dissolved in hexane-ethyl acetate (3:1) mixture and the solution filtered through silica gel to remove some polar material. After evaporation of the solvent, the oily product N-[[5-(heptafluoropropyl)-6-methoxy-1-naphthalenyl]thioxomethyl]-N-methylglycine, methyl ester, solidified on scratching (1.07 g). m.p. 107—109°C. ms: m/e 471 ($M^+$), 440, 412, 369, 207, 102. nmr ($CDCl_3$): δ 3.0 (s, 3H, N—$CH_3$); δ 3.85 (s, 3H, $OCH_3$); δ 3.92 (s, 3H, $OCH_3$); δ 4.93 (q, 2H, $CH_2$); δ 7.1—8.4 (m, 5H, $H_{ar}$).

b) To a solution of the N-[[5-(heptafluoropropyl)-6-methoxy-1-naphthalenyl]thioxomethyl]-N-methylglycine, methyl ester (1.07 g, 2.27 mmol) in 2-methoxy ethanol (15 ml) was added 2N aqueous sodium hydroxide (2.27 ml) at 0°C under nitrogen atmosphere. The mixture was stirred at room temperature overnight, cooled to 0°C, neutralized (pH 7—8) with aqueous 1N hydrochloric acid and evaporated to dryness. The residue was triturated with water, acidified to pH 3 and the product extracted with ethyl acetate, the extract washed repeatedly with water, dried over anhydrous magnesium sulfate and the solvent evaporated. The oily residue crystallized from chloroform-hexane yielded 484 mg of N-[[5-(heptafluoropropyl)-6-methoxy-1-naphthalenyl]thioxomethyl]-N-methylglycine. m.p. 162—163°C. Anal. Calcd: C, 47.27%, H, 3.09% N, 3.06%. Found: C, 47.03% H, 3.08% N, 3.03%. ir ($CHCl_3$): 2950 (broad), 1722, 1760 (infl.), 1110 $cm^{-1}$ uv, λmax (ε): 338 (4210), 228 (48,350). nmr ($CDCl_3$): δ 3.05 (s, 3H, N—$CH_3$); δ 3.93 (s, 3H, O—$CH_3$); δ 4.6 and 5.4 (2d, 2H, N—$CH_2$, J=17); δ 7.6 (m, 5H, $H_{ar}$); δ 8.9 (b, 1H, COOH). ms: m/e 457 ($M^+$), 456, 412, 338, 369, 207, 169, 69.

Example 5

N-[[6-methoxy-5-(tridecafluorohexyl)-1-naphthalenyl]-N-methylglycine (VIII, n = 5)

a) The 6-methoxy-5-iodo-1-naphthylene carboxylic acid, methyl ester (10 g, 0.0292 mol), 1-iodotridecafluoro-hexane (26.05 g, 0.0584 mol), activated copper powder (5.6 g) and dry, distilled dimethylformamide (30 ml) were heated to 140°C with stirring in a pressure bottle for 4 hours. The mixture was cooled, copper removed by filtration, the solvent evaporated, the residue dissolved in ethyl acetate and the solution washed with brine. Drying over anhydrous magnesium sulfate and evaporation, gave 15.6 g of the product, 6-methoxy-5-(tridecafluorohexyl)-1-naphthalene carboxylic acid methyl ester, which was used in the next step without further purification. A small amount (400 mg) was purified by column chromatography ($SiO_2$, hexane-ethyl acetate) for spectral identification (429 mg of pure material). ms: m/e 534, 515, 503, 265, 119, 69, nmr ($CDCl_3$): δ 4.05 (s, 6H, 2 $OCH_3$); δ 7.3—9.3 (m, 5H, $H_{ar}$).

b) To a solution of 6-methoxy-5-(tridecafluorohexyl)-1-naphthalene carboxylic acid methyl ester (0.919 g, 17.2 mmol) in methanol (10 ml) and 2-methoxy ethanol (3 ml) was added aqueous 4N sodium hydroxide (0.86 ml). The mixture was stirred at room temperature for 30 minutes and then refluxed for 2 hours. The solvent was evaporated, the residue triturated with water, the neutral material extracted with ethyl acetate and the aqueous layer separated and acidified with aqueous 1N hydrochloric acid to pH 3. The product was extracted with ethyl acetate, the extract washed with water, dried over anhydrous magnesium sulfate and evaporated (745 mg). The product, 6-methoxy-5-(tridecafluorohexyl)-1-naphthalene carboxylic acid, was used in the next stage without further purification. ms: m/e 520 ($M^+$), 328, 251. nmr (DMSO): δ 4.0 (s, 3H, $OCH_3$); δ 7.5—8.5 (m, 4H, $H_{ar}$); δ 9.2 (d, 1H, $H_{ar}$, J=10).

c) 6-Methoxy-5-(tridecafluorohexyl)-1-naphthalene carboxylic acid (9.8 g, 0.0188 mol), 1-hydroxybenzotriazole (3.82 g, 0.0283 mol), dicyclohexylcarbodiimide (4.66 g, 0.0226 mol) and dry, distilled dimethylformamide (70 ml) were stirred at room temperature for 1 hour. A solution of sarcosine methyl ester hydrochloride (5.26 g, 0.0377 mol) in dimethylformamide (50 ml) and N-ethylmorpholine (4.82 ml) was added to the above reaction mixture. Stirring was continued for 3 hours. Dicyclohexylurea was removed by filtration, washed with dimethylformamide on the filter, the combined filtrates were evaporated and the residue dissolved in ethyl acetate. The solution was washed with 1N aqueous hydrochloric acid, water, saturated sodium bicarbonate, water and dried over anhydrous magnesium sulfate. Evaporation of the solvent gave 10 g of crude N-[[6-methoxy-5-(tridecafluorohexyl)-1-naphthalenyl]carbonyl]-N-methylglycine, methyl ester, which was purified by chromatography by using Waters Prep LC 500A instrument with ethyl acetate-hexane (1:1) system. Yield: 5.15 g. ms: m/e 605 ($M^+$), 503, 336, 206. nmr (DMSO): δ 2.75 and 3.1 (2s, 3H, N—$CH_3$, rotamers); 3.5 and 3.75 (2s, 3H, O—$CH_3$, rotamers); δ 4.0 (s, 3H, $OCH_3$) δ 4.0—4.4 (b, 2H, $CH_2$); 7.2—8.4 (m, 5H, $H_{ar}$).

d) To a solution of N-[[6-methoxy-5-(tridecafluorohexyl)-1-naphthalenyl]carbonyl]-N-methylglycine, methyl ester (1.5 g, 2.48 mmol) in 2-methoxy-ethanol (20 ml) was added 4N aqueous sodium hydroxide (1.24 ml) at 0°C under nitrogen atmosphere. Stirring was continued for 2 hours at room temperature. The reaction mixture was diluted with water, acidified with 1N aqueous hydrochloric acid to pH 3 and extracted with ether. The extract was washed with water, dried over anhydrous magnesium sulfate and evaporated. The pure product N-[[6-methoxy-5-(tridecafluorohexyl)-1-naphthalenyl]-N-methylglycine (731 mg) was obtained by crystallization from ethanol-water. Additional product (492 mg) was isolated from the mother liquor. Anal. Calcd: C, 42.65% H, 2.39% N, 2.37%. Found: C, 42.48% H, 2.43% N, 2.42%. ir (nujol): 2500, 1700, 1600, 1580, 1500, 920 $cm^{-1}$. uv, λ max (ε): 337 (3490), 324 (3250), 297 (6150), 286 (6450), 227 (52,690). nmr (DMSO): δ 2.75 and 3.13 (2s, 3H, N—$CH_3$, rotamers); δ 4.00 (s, 3H, $OCH_3$); δ 4.23 (b, 2H, $CH_2$); δ 7.2—8.4 (m, 5H, $H_{ar}$); δ 12.8 (b, 1H, $NH^+$). ms: m/e 591 ($M^+$), 573, 547, 504, 322.

Example 6

N-[[6-Methoxy-5-(tridecafluorohexyl)-1-naphthalenyl]thioxomethyl]-N-methylglycine (VI n = 5)

a) N-[[6-methoxy-5-(tridecafluorohexyl)-1-naphthalenyl]carbonyl-N-methylglycine, methyl ester (100 mg, 0.165 mmol), prepared as in Example 5, phosphorus pentasulfide (67 mg) and dry pyridine (2 ml) were refluxed for 2 hours. After stirring overnight at room temperature, the mixture was refluxed for 2 additional hours, poured into water and extracted with ether. The ether extract was washed with 1N aqueous hydrochloric acid, water, saturated sodium bicarbonate and brine. After drying over anhydrous magnesium sulfate, the solvent was evaporated giving 92.8 mg of the crude N-[[6-methoxy-5-(tridecafluorohexyl)-1-naphthalenyl]thioxomethyl]-N-methylglycine, methyl ester, which was used in the next preparation without further purification. ms: m/e 622 $(M + H)^+$, 562, 519, 503, 352. nmr ($CDCl_3$): δ 2.84 and 3.04 (2s, 3H, N—$CH_3$, rotamers); δ 3.82 and 3.95 (2s, 3H, O—$CH_3$, rotamers) δ 3.4 and 5.5 (2d, 2H, $CH_2$, J=17); δ 7.3 (m, 3H, $H_{ar}$); δ 8.2 (m, 2H, $H_{ar}$).

b) To a solution of N-[[6-methoxy-5-(tridecafluorohexyl)-1-naphthalenyl]thioxomethyl]-N-methylglycine, methyl ester (2.53 g, 4.07 mmol) in 2-methoxy-ethanol (20 ml) was added aqueous 4N sodium hydroxide (2 ml) with stirring under nitrogen atmosphere at 0°C. Stirring was continued for 2 hours at room temperature. The mixture was diluted with water, neutral material removed by extraction with ether, the aqueous layer acidified to pH 3 and the product extracted with ether. The extract was washed with water and dried over anhydrous magnesium sulfate. Evaporation and trituration with hexane gave 818 mg of N-[[6-methoxy-5-(tridecafluorohexyl)-1-naphthalenyl]thioxomethyl]-N-methylglycine. m.p. 180°C (dec.) Anal. Calcd: C, 41.53% H, 2.32% N, 2.31% Found: C, 39.79% H, 2.34% N, 2.29%. ms: m/e 607 $(M^+)$, 562, 519, 338. ir ($CHCl_3$): 3400, 1720, 1600, 1500, 1450, 1140, 920 $cm^{-1}$. uv, λmax (ε): 307 (4310), 272 (11,600), 228 (44,890). nmr ($CDCl_3$): δ 2.90 (s, 3H, N—$CH_3$); δ 3.94 (s, 3H, $OCH_3$); δ 4.25 and 5.25 (2d, 2H, $CH_2$, J=16); δ 7.2 (m, 1H, $H_{ar}$); δ 7.55 (m, 2H, $H_{ar}$); δ 7.9 (j, 1H, $H_{ar}$); δ 8.55 (m, 1H, $H_{ar}$).

**Claims for the Contracting States: BE CH DE FR GB IT LI LU NL SE**

1. A compound of formula I

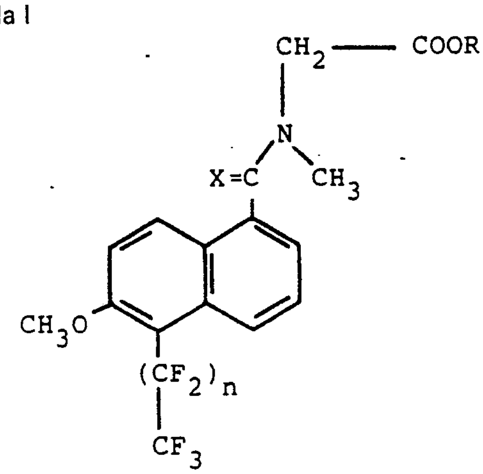

(I)

wherein n is an integer from 1 to 5, X is oxygen or sulphur and R is hydrogen or a $C_{1-4}$ alkyl group, or a therapeutically acceptable salt of the compound of formula I where R is hydrogen with an organic or inorganic base.

2. A compound as claimed in Claim 1 wherein R is hydrogen.

3. A compound as claimed in Claim 1 or Claim 2 wherein n is 2.

4. A compound of formula I as claimed in Claim 1 which is
N-[[6-methoxy-5-(pentafluoroethyl)-1-naphthalenyl]thioxomethyl]-N-methylglycine;
N-[[6-methoxy-5-(pentafluoroethyl)-1-naphthalenyl]carbonyl]-N-methylglycine;
N-[[6-methoxy-5-(tridecafluorohexyl)-1-naphthalenyl]thioxomethyl]-N-methylglycine; or
N-[[6-methoxy-5-(tridecafluorohexyl)-1-naphthalenyl]carbonyl]-N-methylglycine;
or a therapeutically acceptable salt thereof with an organic or inorganic base.

5. N-[[5-(Heptafluoropropyl)-6-methoxy-1-naphthalenyl]thioxomethyl]-N-methylglycine or a therapeutically acceptable salt thereof with an organic or inorganic base.

6. N-[[5-(Heptafluoropropyl)-6-methoxy-1-naphthalenyl]carbonyl]-N-methylglycine or a therapeutically acceptable salt thereof with an organic or inorganic base.

7. The corresponding methyl ester of a compound of formula I as claimed in any one of Claims 4 to 6.

8. A pharmaceutical composition comprising a compound of formula I as claimed in any one of Claims 1 to 7 and a pharmaceutically acceptable carrier.

9. A compound of formula I as defined in any one of Claims 1 to 7 for use as a pharmaceutical.

10. A process for preparing a compound of formula I as defined in Claim 1 or a therapeutically acceptable salt with an organic or inorganic base of the compound of formula I wherein R is hydrogen

which comprises one of the following:

(i) hydrolysing a compound of formula IX

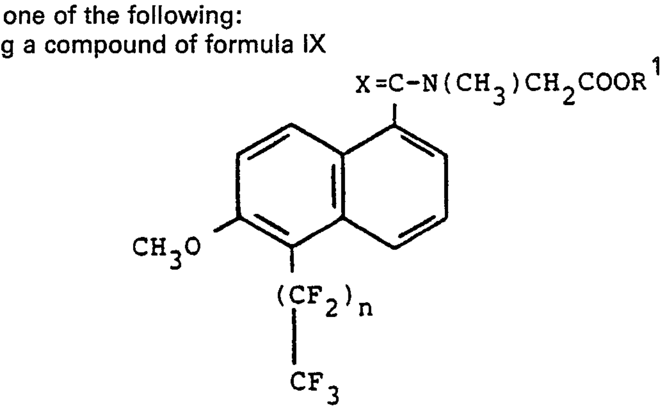

IX

where $COOR^1$ is an ester function, and n and X are as defined in Claim 1 to give a compound of formula I wherein R is hydrogen;

(ii) reacting a compound of formula Va

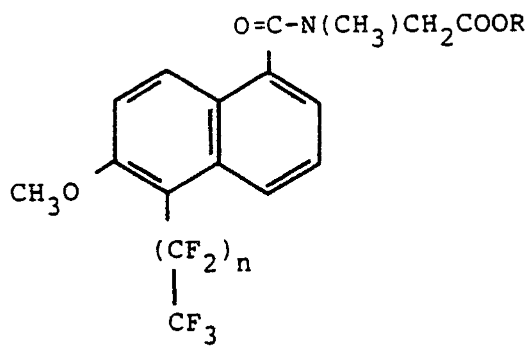

Va

wherein R and n are as defined in Claim 1 with phosphorus pentasulphide to give a compound of formula I wherein X is sulphur;

(iii) coupling a compound of formula

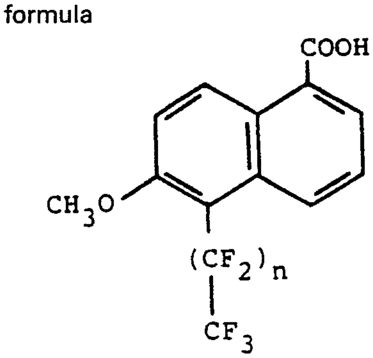

IV

wherein n is defined in Claim 1, with a compound of formula

$$HN(CH_3)CH_2COO(C_{1-4}\ alkyl) \qquad (X)$$

to give a compound of formula I wherein R is $C_{1-4}$ alkyl,

(iv) reacting a compound of formula

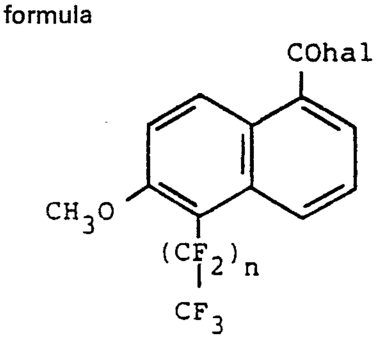

(XI)

in which formula n is as defined in Claim 1 and hal represents a chlorine or bromine with N-methylglycine to give a compound of formula I wherein X is oxygen and R is hydrogen;

(v) perfluoroalkylating a compound of formula

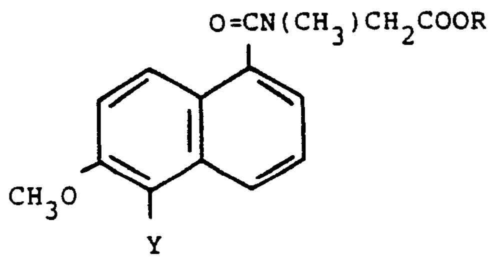

(XII)

wherein Y represents chlorine, bromine or iodine and R is as defined in Claim 1 with an alkylating agent containing the group $CF_3(CF_2)_n$— to give a compound of formula I wherein X = oxygen or

(vi) reacting a compound of formula I wherein R is H with an organic or inorganic base to form a therapeutically acceptable salt or

(vii) acidifying a salt of a compound of formula I to give a compound of formula I wherein R is hydrogen.

11. A process of preparing a compound of claim 1 wherein X is O which comprises perfluoroalkylating 6-methoxy-5-iodo-1-naphthalene carboxylic acid, methyl ester to form the corresponding perfluoroalkyl compound, hydrolyzing said perfluoroalkyl compound to the corresponding carboxylic acid, coupling said acid with sarcosine methylester to form the corresponding substituted N-methylglycine, methyl ester, hydrolyzing said substituted N-methylglycine, methyl ester to the corresponding substituted N-methylglycine.

12. A modification of the process of Claim 11, wherein said N-methylglycine, methyl ester is reacted with $P_2S_5$ to form the corresponding substituted thioxomethyl-N-methylglycine, methyl ester and hydrolyzing said substituted thioxomethyl-N-methylglycine, methyl ester to the corresponding substituted thioxomethyl-N-methylglycine.

**Claims for the Contracting State: AT**

1. A process for preparing a compound of formula I

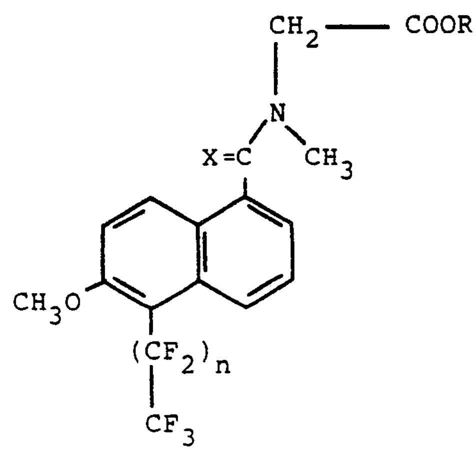

(I)

wherein n is an integer from 1 to 5, X is oxygen or sulphur and R is hydrogen or a $C_{1-4}$ alkyl group, or a therapeutically acceptable salt of the compound of formula I where R is hydrogen with an organic or inorganic base, which comprises

(i) hydrolysing a compound of formula IX

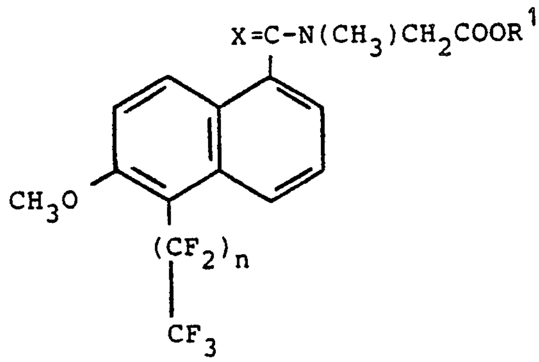

IX

where $COOR^1$ is an ester function, and n and X are as defined above to give a compound of formula I wherein R is hydrogen; or

(ii) reacting a compound of formula Va

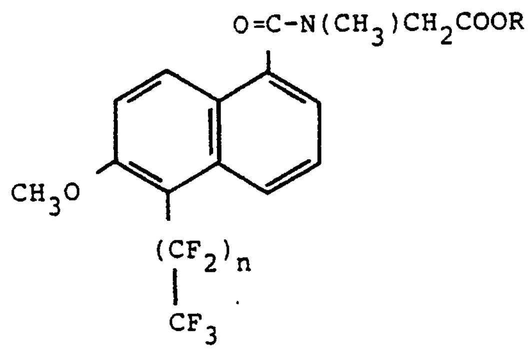

Va

wherein R and n are as defined above with phosphorus pentasulphide to give a compound of formula I wherein X is sulphur; or

(iii) coupling a compound of formula

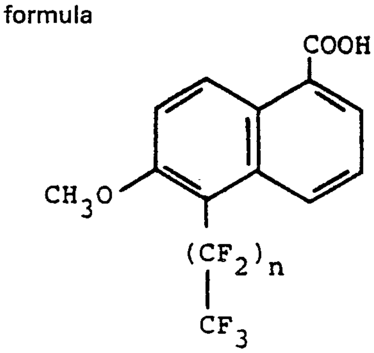

IV

wherein n is as defined above, with a compound of formula

$$HN(CH_3)CH_2COO(C_{1-4}\ alkyl) \quad (X)$$

to give a compound of formula I wherein R is $C_{1-4}$ alkyl; or

(iv) reacting a compound of formula

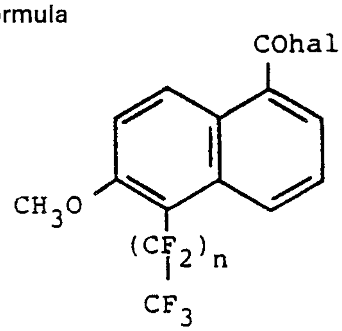

(XI)

in which formula n is as defined above and hal represents a chlorine or bromine with N-methylglycine to give a compound of formula I wherein X is oxygen and R is hydrogen; or

(v) perfluoroalkylating a compound of formula

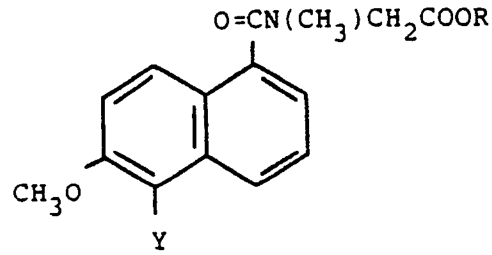

(XII)

wherein Y represents chlorine, bromine or iodine and R is as defined above with an alkylating agent containing the group $CF_3(CF_2)_n$— to give a compound of formula I wherein X = oxygen; or

(vi) reacting a compound of formula I wherein R is H with an organic or inorganic base to form a therapeutically acceptable salt; or

(vii) acidifying a salt of a compound of formula I to give a compound of formula I wherein R is hydrogen.

2. A process of preparing a compound of claim 1 wherein X is O which comprises perfluoroalkylating 6-methoxy-5-iodo-1-naphthalene carboxylic acid, methyl ester to form the corresponding perfluoroalkyl compound, hydrolyzing said perfluoroalkyl compound to the corresponding carboxylic acid, coupling said acid with sarcosine methylester to form the corresponding substituted N-methylglycine, methyl ester, hydrolyzing said substituted N-methylglycine, methyl ester to the corresponding substituted N-methylglycine.

3. A modification of the process of Claim 2, wherein said N-methylglycine, methyl ester is reacted with $P_2S_5$ to form the corresponding substituted thioxomethyl-N-methylglycine, methyl ester and hydrolyzing said substituted thioxomethyl-N-methylglycine, methyl ester to the corresponding substituted thioxomethyl-N-methylglycine.

4. A process as claimed in Claim 1, 2 or 3 wherein R in the compound prepared is hydrogen.

5. A process as claimed in any one of Claims 1 to 4 wherein n is 2.

6. A process as claimed in any one of Claims 1 to 3 in which the compound prepared is
N-[[6-methoxy-5-(pentafluoroethyl)-1-naphthalenyl]thioxomethyl]-N-methylglycine; or
N-[[6-methoxy-5-(pentafluoroethyl)-1-naphthalenyl]carbonyl]-N-methylglycine; or
N-[[6-methoxy-5-(tridecafluorohexyl)-1-naphthalenyl]thioxomethyl]-N-methylglycine; or
N-[[6-methoxy-5-(tridecafluorohexyl)-1-naphthalenyl]carbonyl]-N-methylglycine;
or a therapeutically acceptable salt thereof with an organic or inorganic base.

7. A process as claimed in Claim 1 or Claim 3 in which the compound prepared is N-[[5-(heptafluoropropyl)-6-methoxy-1-naphthalenyl]-thioxomethyl]-N-methylglycine or a therapeutically acceptable salt thereof with an organic or inorganic base.

8. A process as claimed in Claim 1 or Claim 2 in which the compound prepared is N-[[5-(heptafluoropropyl)-6-methoxy-1-naphthalenyl]carbonyl]-N-methylglycine or a therapeutically acceptable salt thereof with an organic or inorganic base.

9. A process as claimed in Claim 1 in which the compound prepared is the corresponding methyl ester of a compound of formula I as prepared in any one of Claims 6 to 8.

10. A process for preparing a pharmaceutical composition which comprises bringing a compound of formula I as defined in Claim 1 or a therapeutically acceptable salt thereof into association with a pharmaceutically acceptable carrier.

**Patentansprüche für die Vertragsstaaten: BE CH DE FR GB IT LI LU NL SE**

1. Verbindung der Formel (I)

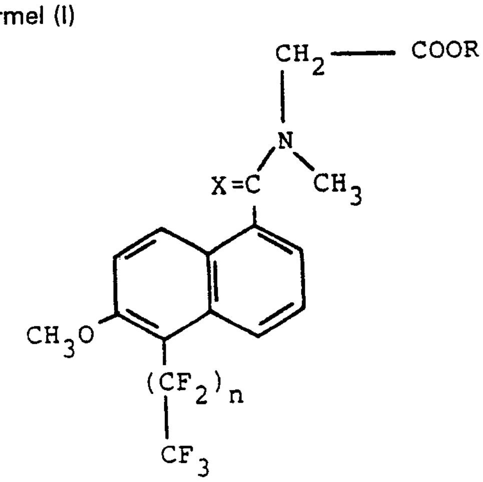

(I)

worin n eine ganze Zahl von 1 bis 5 ist, X Sauerstoff oder Schwefel bedeutet und R Wasserstoff oder eine $C_{1-4}$-Alkylgruppe darstellt, oder ein therapeutisch annehmbares Salz der Verbindung der Formel (I), worin R Wasserstoff ist, mit einer organischen oder anorganischen Base.

2. Verbindung, wie in Anspruch 1 beansprucht, worin R Wasserstoff ist.

3. Verbindung, wie in Anspruch 1 oder 2 beansprucht, worin n 2 ist.

4. Verbindung der Formel (I), wie in Anspruch 1 beansprucht, die
N-[[6-Methoxy-5-(pentafluoräthyl)-1-naphthalenyl]-thioxomethyl]-N-methylglycin,
N-[[6-Methoxy-5-(pentafluoräthyl)-1-naphthalenyl]-carbonyl]-N-methylglycin,
N-[[6-Methoxy-5-(tridecafluorhexyl)-1-naphthalenyl]-thioxomethyl]-N-methylglycin oder
N-[[6-Methoxy-5-(tridecafluorhexyl)-1-naphthalenyl]-carbonyl]-N-methylglycin
oder ein therapeutisch annehmbares Salz hievon mit einer organischen oder anorganischen Base ist.

5. N-[[5-(Heptafluorpropyl)-6-methoxy-1-naphthalenyl]thioxomethyl]-N-methylglycin oder ein therapeutisch annehmbares Salz hievon mit einer organischen oder anorganischen Base.

6. N-[[5-(Heptafluorpropyl)-6-methoxy-1-naphthalenyl]carbonyl]-N-methylglycin oder ein therapeutisch annehmbares Salz hievon mit einer organischen oder anorganischen Base.

7. Der entsprechende Methylester einer Verbindung der Formel (I), wie in einem der Ansprüche 4 bis 6 beansprucht.

8. Pharmazeutische Zusammensetzung umfassend eine Verbindung der Formel (I), wie in einem der Ansprüche 1 bis 7 beansprucht, und einen pharmazeutisch annehmbaren Träger.

9. Verbindung der Formel (I), wie in einem der Ansprüche 1 bis 7 definiert, zur Verwendung als Pharmazeutikum.

10. Verfahren zum Herstellen einer Verbindung der Formel (I), wie in Anspruch 1 definiert, oder eines therapeutisch annehmbaren Salzes mit einer organischen oder anorganischen Base der Verbindung der Formel (I) , worin R Wasserstoff ist, welches eines der folgenden umfaßt:

(i) Hydrolysieren einer Verbindung der Formel (IX)

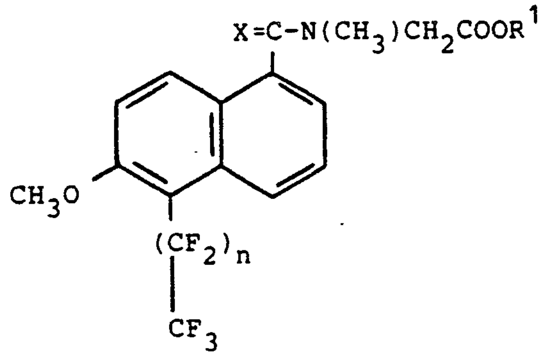

IX

worin $COOR^1$ eine Esterfunktion ist und n und X wie in Anspruch 1 definiert sind, zu einer Verbindung der Formel (I), worin R Wasserstoff ist;

(ii) Umsetzen einer Verbindung der Formel (Va)

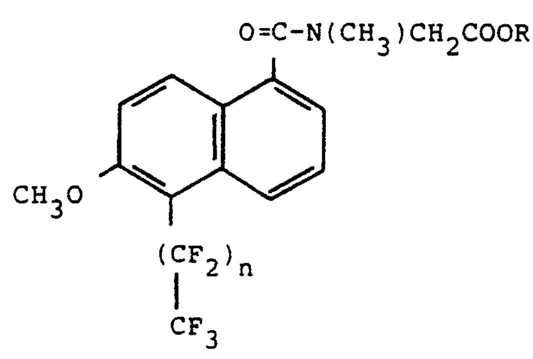

Va

worin R und n wie in Anspruch 1 definiert sind, mit Phosphorpentasulfid zu einer Verbindung der Formel (I), worin X Schwefel ist;

(iii) Koppeln einer Verbindung der Formel

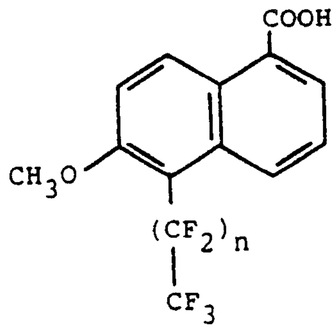

IV

worin n wie in Anspruch 1 definiert ist, mit einer Verbindung der Formel

$HN(CH_3)CH_2COO(C_{1-4}\text{-Alkyl})$ (X)

zu einer Verbindung der Formel (I), worin R $C_{1-4}$-Alkyl ist;

(iv) Umsetzung einer Verbindung der Formel

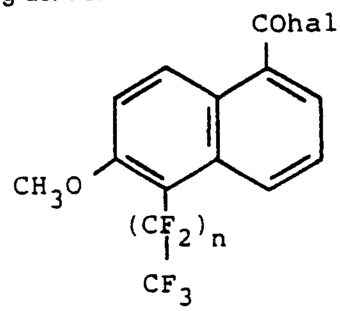

(XI)

in welcher Formel n wie in Anspruch 1 definiert ist und hal Chlor oder Brom bedeutet, mit N-Methylglycin zu einer Verbindung der Formel (I), worin X Sauerstoff und R Wasserstoff sind;

(v) Perfluoralkylieren einer Verbindung der Formel

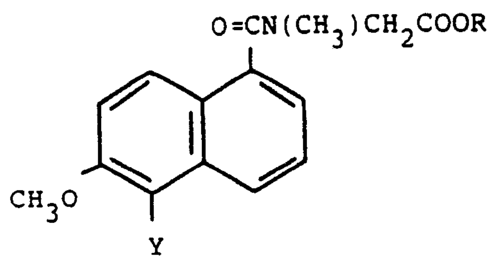

(XII)

worin Y Chlor, Brom oder Jod bedeutet und R wie in Anspruch 1 definiert ist, mit einem Alkylierungsmittel, das die Gruppe $CF_3(CF_2)_n$— enthält, zu einer Verbindung der Formel (I), worin X Sauerstoff ist oder

(vi) Umsetzen einer Verbindung der Formel (I), worin R H bedeutet, mit einer organischen oder anorganischen Base zur Bildung eines therapeutisch annehmbaren Salzes oder

(vii) Ansäuern eines Salzes einer Verbindung der Formel (I), wobei eine Verbindung der Formel (I) erhalten wird, worin R Wasserstoff ist.

11. Verfahren zum Herstellen einer Verbindung von Anspruch 1, worin X O bedeutet, welches das perfluoralkylieren des 6-Methoxy-5-jod-1-naphthalincarbonsäure-methylesters unter Bildung der entsprechenden Perfluoralkylverbindung, das Hydrolysieren dieser Perfluoralkylverbindung zur entsprechenden Carbonsäure, das Koppeln dieser Säure mit Sarcosinmethylester unter Bildung des entsprechenden substituierten N-Methylglycinmethylesters und das Hydrolysieren dieses substituierten N-Methylglycinmethylesters zum entsprechenden substituierten N-Methylglycin umfaßt.

12. Modifizierung des Verfahrens nach Anspruch 11, worin der N-Methylglycinmethylester mit $P_2S_5$ zum entsprechenden substituierten Thioxomethyl-N-methylglycinmethylester umgesetzt und dieser substituierte Thioxomethyl-N-methylglycinmethylester zum entsprechenden substituierten Thioxomethyl-N-methylglycin hydrolysiert wird.

**Patentansprüche für den Vertragsstaat: AT**

1. Verfahren zum Herstellen einer Verbindung der Formel (I)

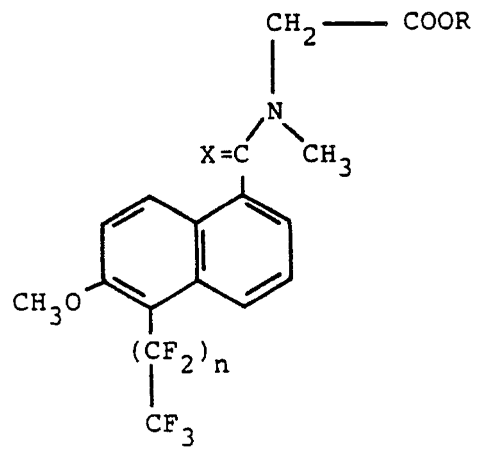

(I)

worin n eine ganze Zahl von 1 bis 5 ist, X Sauerstoff oder Schwefel bedeutet und R Wasserstoff oder eine $C_{1-4}$-Alkylgruppe darstellt, oder eines therapeutisch annehmbaren Salzes der Verbindung der Formel (I), worin R Wasserstoff ist, mit einer organischen oder anorganischen Base, welches umfaßt

(i) Hydrolysieren einer Verbindung der Formel (IX )

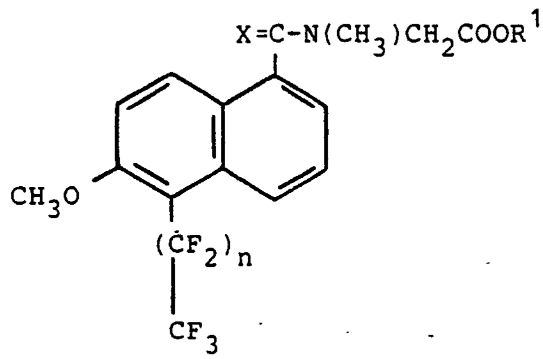

IX

worin $COOR^1$ eine Esterfunktion ist und n und X wie oben definiert sind, zu einer Verbindung der Formel (I), worin R Wasserstoff ist;

(ii) Umsetzen einer Verbindung der Formel (Va)

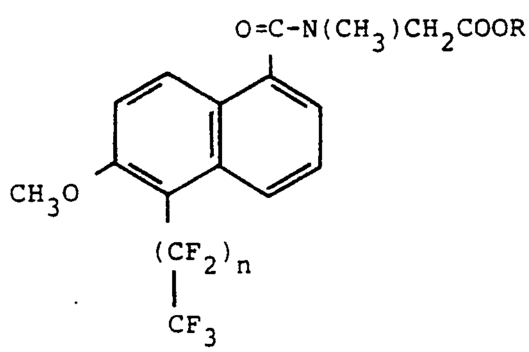

Va

worin R und n wie oben definiert sind, mit Phosphorpentasulfid zu einer Verbindung der Formel (I), worin X Schwefel ist;

(iii) Koppeln einer Verbindung der Formel

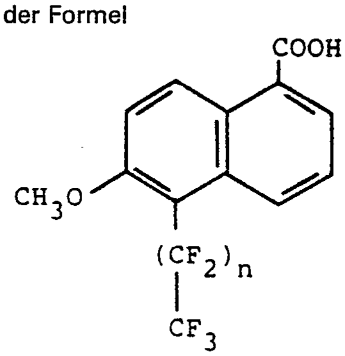

IV

worin n wie oben definiert ist, mit einer Verbindung der Formel

$$HN(CH_3)CH_2COO(C_{1-4}\text{-Alkyl}) \quad (X)$$

zu einer Verbindung der Formel (I), worin R $C_{1-4}$-Alkyl ist;

(iv) Umsetzung einer Verbindung der Formel

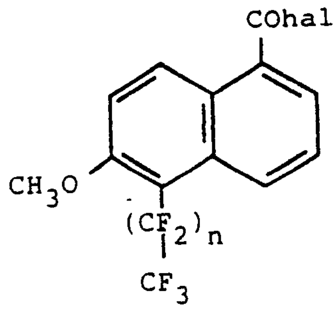

(XI)

in welcher Formel n wie oben definiert ist und hal Chlor oder Brom bedeutet, mit N-Methylglycin zu einer Verbindung der Formel (I), worin X Sauerstoff und R Wasserstoff sind;

(v) Perfluoralkylieren einer Verbindung der Formel

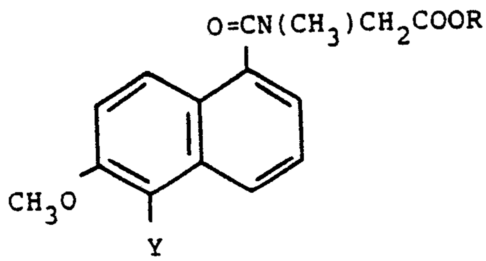

(XII)

worin Y Chlor, Brom oder Jod bedeutet und R wie oben definiert ist, mit einem Alkylierungsmittel, das die Gruppe $CF_3(CF_2)_n$— enthält, zu einer Verbindung der Formel (I), worin X Sauerstoff ist, oder

(vi) Umsetzen einer Verbindung der Formel (I), worin R H bedeutet, mit einer organischen oder anorganischen Base zur Bildung eines therapeutisch annehmbaren Salzes oder

(vii) Ansäuern eines Salzes einer Verbindung der Formel (I), wobei eine Verbindung der Formel (I) erhalten wird, worin R Wasserstoff ist.

2. Verfahren zum Herstellen einer Verbindung von Anspruch 1, worin X O bedeutet, welches das perfluoralkylieren des 6-Methoxy-5-jod-1-naphthalincarbonsäure-methylesters unter Bildung der entsprechenden Perfluoralkylverbindung, das Hydrolysieren dieser Perfluoralkylverbindung zur entsprechenden Carbonsäure, das Koppeln dieser Säure mit Sarcosinmethylester unter Bildung des entsprechenden substituierten N-Methylglycinmethylesters und das Hydrolysieren dieses substituierten N-Methylglycinmethylesters zum entsprechenden substituierten N-Methylglycin umfaßt.

3. Modifizierung des Verfahrens nach Anspruch 2, worin der N-Methylglycinmethylester mit $P_2S_5$ zum entsprechenden substituierten Thioxomethyl-N-methylglycinmethylester umgesetzt und dieser substituierte Thioxomethyl-N-methylglycinmethylester zum entsprechenden substituierten Thioxomethyl-N-methylglycin hydrolysiert wird.

4. Verfahren, wie in Anspruch 1, 2 oder 3 beansprucht, wobei R in der hergestellten Verbindung Wasserstoff ist.

5. Verfahren, wie in einem der Ansprüche 1 bis 4 beansprucht, worin n 2 ist.

6. Verfahren, wie in einem der Ansprüche 1 bis 3 beansprucht, wobei die hergestellte Verbindung
N-[[6-Methoxy-5-(pentafluoräthyl)-1-naphthalenyl]-thioxomethyl]-N-methylglycin,
N-[[6-Methoxy-5-(pentafluoräthyl)-1-naphthalenyl]-carbonyl]-N-methylglycin,
N-[[6-Methoxy-5-(tridecafluorhexyl)-1-naphthalenyl]-thioxomethyl]-N-methylglycin oder
N-[[6-Methoxy-5-(tridecafluorhexyl)-1-naphthalenyl]-carbonyl]-N-methylglycin
oder ein therapeutisch annehmbares Salz hievon mit einer organischen oder anorganischen Base ist.

7. Verfahren, wie in Anspruch 1 oder 3 beansprucht, wobei die hergestellte Verbindung N-[[5-(Heptafluorpropyl)-6-methoxy-1-naphthalenyl]thioxomethyl]-N-methylglycin oder ein therapeutisch annehmbares Salz hievon mit einer organischen oder anorganischen Base.

8. Verfahren, wie in Anspruch 1 oder 2 beansprucht, wobei die hergestellte Verbindung N-[[5-(Heptafluorpropyl)-6-methoxy-1-naphthalenyl]carbonyl]-N-methylglycin oder ein therapeutisch annehmbares Salz hievon mit einer organischen oder anorganischen Base.

9. Verfahren, wie in Anspruch 1 beansprucht, wobei die hergestellte Verbindung der entsprechende Methylester einer Verbindung der Formel (I), hergestellt wie in einem der Ansprüche 6 bis 8 ist.

10. Verfahren zum Herstellen einer pharmazeutischen Zusammensetzung, welches das Vereinigen einer Verbindung der Formel (I), wie in Anspruch 1 definiert, oder eines therapeutisch annehmbaren Salzes hievon mit einem pharmazeutisch annehmbaren Träger umfaßt.

**Revendications pour les Etats contractants: BE CH DE FR GB IT LI LU NL SE**

1. Composé de formule I

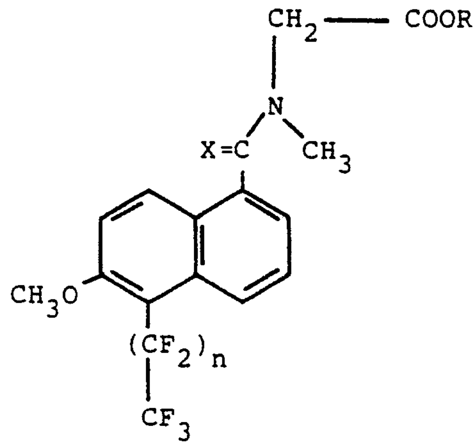

(I)

dans laquelle *n* représente un nombre entier de 1 à 5, X représente l'oxygène ou le soufre et R représente l'hydrogène ou un group alkyle en $C_1$ à $C_4$, ou un sel thérapeutiquement acceptable du composé de formule I, dans laquelle R représente l'hydrogène, formé avec une base organique ou inorganique.

2. Composé suivant la revendication 1, dans lequel R représente l'hydrogène.

3. Composé suivant la revendication 1 ou la revendication 2, dans lequel *n* a la valeur 2.

4. Composé de formule I suivant la revendication 1, qui est
la N-[[6-méthoxy-5-(pentafluoréthyl)-1-naphtalényl]thioxométhyl]-N-méthylglycine;
la N-[[6-méthoxy-5-(pentafluoréthyl)-1-naphtalényl]carbonyl]-N-méthylglycine;
la N-[[6-méthoxy-5-(tridécafluorhexyl)-1-naphtalényl]thioxométhyl]-N-méthylglycine; ou
la N-[[6-méthoxy-5-(tridécafluorhexyl)-1-naphtalényl]carbonyl]-N-méthylglycine;
ou un de ses sels thérapeutiquement acceptables, formé avec une base organique ou inorganique.

5. N-[[5-(heptafluoropropyl)-6-méthoxy-1-naphtalényl]thioxométhyl]-N-méthylglycine, ou un de ses sels thérapeutiquement acceptables, formé avec une base organique ou inorganique.

6. N-[[5-(heptafluoropropyl)-6-méthoxy-1-naphtalényl]carbonyl]-N-méthylglycine, ou un de ses sels thérapeutiquement acceptables, formé avec une base organique ou inorganique.

7. Ester méthylique correspondant à un composé de formule I suivant l'une quelconque des revendications 4 à 6.

8. Composition pharmaceutique comprenant un composé de formule I suivant l'une quelconque des revendications 1 à 7 et un support pharmaceutiquement acceptable.

9. Composé de formule I suivant l'une quelconque des revendications 1 à 7, destiné à être utilisé comme substance pharmacetique.

10. Procédé de préparation d'un composé de formule I suivant la revendication 1 ou d'un sel thérapeutiquement acceptable, formé avec une base organique ou inorganique, du composé de formule I dans laquelle R représente l'hydrogène, qui consiste à effectuer l'une des réactions suivantes:
(i) hydrolyse d'un composé de formule IX

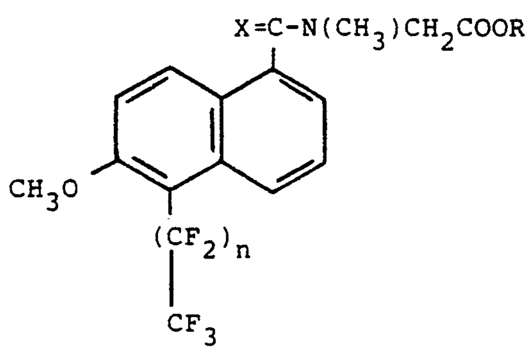

IX

dans laquelle $COOR^1$ représente une fonction ester et *n* et X répondent aux définitions suivant la revendication 1, pour donner un composé de formule I dans laquelle R représente l'hydrogène;

(ii) réaction d'un composé de formule Va

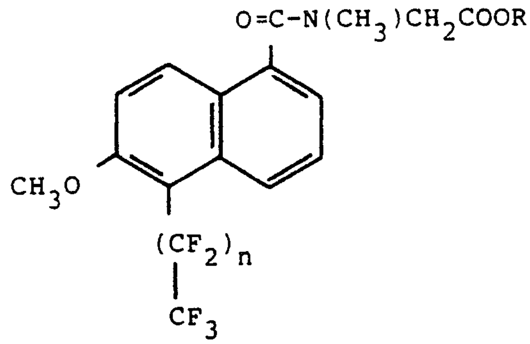

Va

dans laquelle R et *n* répondent aux définitions suivant la revendication 1 avec le pentasulfure de phosphore pour donner un composé de formule I dans laquelle X représente le soufre;

(iii) couplage d'un composé de formule

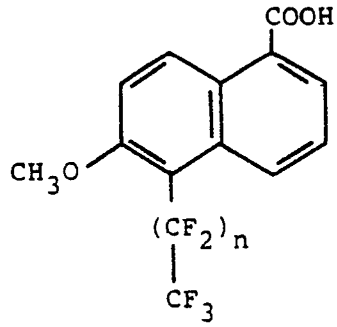

IV

dans laquelle *n* rèpond à la définition suivant la revendication 1, avec un composé de formule

$$HN(CH_3)CH_2COO(\text{alkyle en } C_1 \text{ à } C_4) \qquad (X)$$

pour donner un composé de formule I dans laquelle R représente un groupe alkyle en $C_1$ à $C_4$,

(iv) réaction d'un composé de formule

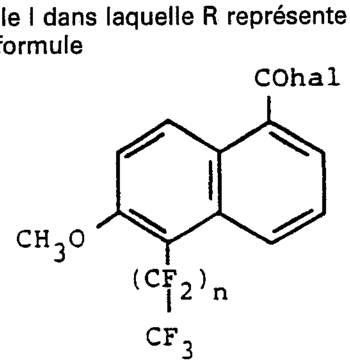

(XI)

dans laquelle *n* répond à la définition suivant la revendication 1 et hal représente un atome de chlore ou de brome, avec la N-méthylglycine pour donner un composé de formule I dans laquelle X représente l'oxygène et R représente l'hydrogène;

(v) perfluoralkylation d'un composé de formule

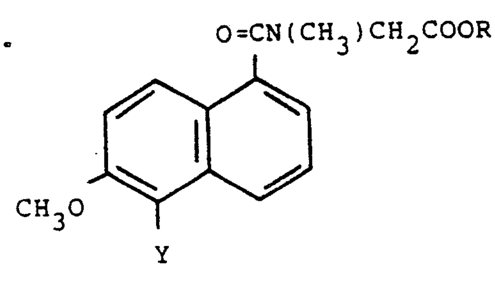

(XII)

dans laquelle Y représente le chlore, le brome ou l'iode et R répond à la définition suivant la revendication 1, avec un agent d'alkylation contenant le groupe $CF_3(CF_2)_n$— pour donner un composé de formule I dans laquelle X représente l'oxygène, ou

(vi) réaction d'un composé de formule I dans laquelle R représente H avec une base organique ou inorganique pour former un sel thérapeutiquement acceptable, ou bien

(vii) acidification d'un sel d'un composé de formule I pour donner un composé de formule I dans laquelle R représente l'hydrogène.

11. Procédé de préparation d'un composé suivant la revendication 1 dans lequel X représente O, qui consiste à effectuer une perfluoralkylation de l'ester méthylique de l'acide 6-méthoxy-5-iodo-1-naphtalène-carboxylique pour former le composé perfluoralkylique correspondant, à hydrolyser ledit composé perfluoralkylique en l'acide carboxylique correspondant, à coupler ledit acide à l'ester méthylique de sarcosine pour former l'ester méthylique de N-méthylglycine substituée correspondant, à hydrolyser ledit ester méthylique de N-méthylglycine substituée en la N-méthylglycine substituée correspondante.

12. Modification du procédé suivant la revendication 11, dans laquelle l'ester méthylique de N-méthylglycine est amené à réagir avec $P_2S_5$ pour former l'ester méthylique de thioxométhyl-N-méthylglycine substituée correspondant et l'ester méthylique de thioxométhyl-N-méthylglycine substituée est hydrolysé en la thioxométhyl-N-méthylglycine substituée correspondante.

**Revendications pour l'Etat contractant: AT**

1. Procédé de préparation d'un composé de formule I

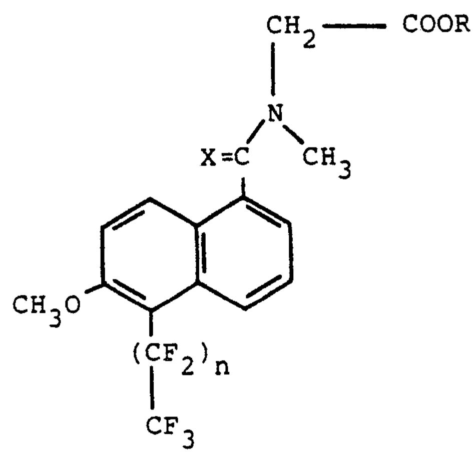

(I)

dans laquelle *n* est un nombre entier de 1 à 5, X représente l'oxygène ou le soufre et.R représente l'hydrogène ou un groupe alkyle en $C_1$ à $C_4$, ou d'un sel thérapeutiquement acceptable du composé de formule I dans laquelle R représente l'hydrogène, formé avec une base organique ou inorganique, qui consiste

(i) à hydrolyser un composé de formule IX

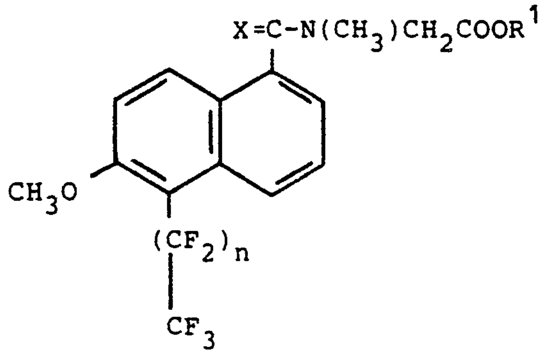

IX

dans laquelle $COOR^1$ représente une fonction ester, et *n* et X répondent aux définitions précitées, pour donner un composé de formule I dans laquelle R représente l'hydrogène; ou

(ii) à faire réagir un composé de formule Va

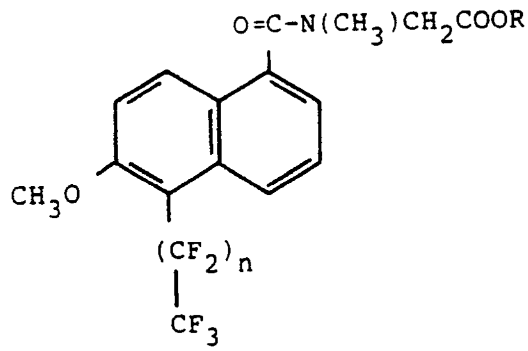

Va

dans laquelle R et *n* répondent aux définitions précitées, avec le pentasulfure de phosphore pour donner un composé de formule I dans laquelle X représente le soufre; ou

(iii) à coupler un composé de formule

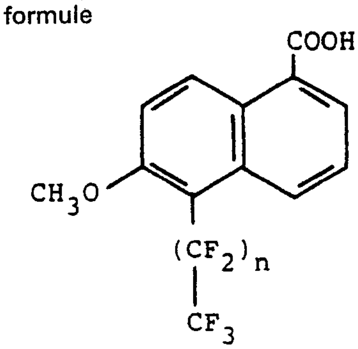

IV

dans laquelle *n* répond à la définition précitée, avec un composé de formule

$$HN(CH_3)CH_2COO(\text{alkyle en } C_1 \text{ à } C_4) \qquad (X)$$

pour donner un composé de formule I dans laquelle R représente un groupe alkyle en $C_1$ à $C_4$, ou

(iv) à faire réagir un composé de formule

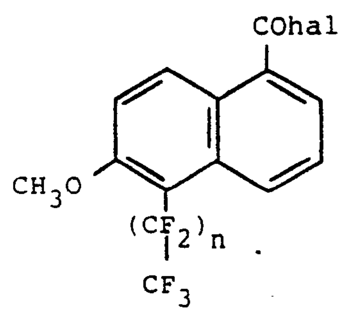

(XI)

dans laquelle *n* répond à la définition précitée, et hal représente un atome de chlore ou de brome, avec la N-méthylglycine pour donner un composé de formule I dans laquelle X représente l'oxygène et R représente l'hydrogène; ou

(v) à effectuer la perfluoralkylation d'un composé de formule

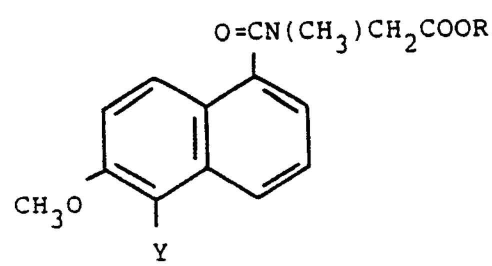

(XII)

dans laquelle Y représente le chlore, le brome ou l'iode et R répond à la définition précitée, avec un agent d'alkylation contenant le groupe $CF_3(CF_2)_n$— pour donner un composé de formule I dans laquelle X représente l'oxygène, ou

(vi) à faire réagir un composé de formule I dans laquelle R représente H avec une base organique ou inorganique pour former un sel thérapeutiquement acceptable; ou bien

(vii) à acidifier un sel d'un composé de formule I pour donner un composé de formule I dans laquelle R représente l'hydrogène.

2. Procédé de préparation d'un composé suivant la revendication 1, dans lequel X représente O, qui consiste à effectuer une perfluoralkylation de l'ester méthylique de l'acide 6-méthoxy-5-iodo-1-naphtalène-carboxylique pour former le composé perfluoralkylique correspondant, à hydrolyser ledit composé perfluoralkylique en ledit acide carboxylique correspondant, à coupler ledit acide avec l'ester méthylique de sarcosine pour former l'ester méthylique de N-méthylglycine substituée correspondant, à hydrolyser ledit ester méthylique de N-méthylglycine substituée en la N-méthylglycine substituée correspondante.

3. Modification du procédé suivant la revendication 2, dans laquelle l'ester méthylique de N-méthylglycine est amené à réagir avec $P_2S_5$ pour former l'ester méthylique de thioxométhyl-N-méthylglycine substituée correspondant et ledit ester méthylique de thioxométhyl-N-méthylglycine substituée est hydrolysé en la thioxométhyl-N-méthylglycine substituée correspondante.

4. Procédé suivant la revendication 1, 2 ou 3, dans lequel R, dans le composé préparé, représente l'hydrogène.

5. Procédé suivant l'une quelconque des revendications 1 à 4, dans lequel *n* a la valeur 2.

6. Procédé suivant l'une quelconque des revendications 1 à 3, dans lequel le composé préparé est
la N-[[6-méthoxy-5-(pentafluoréthyl)-1-naphtalényl]thioxométhyl]-N-méthylglycine; ou
la N-[[6-méthoxy-5-(pentafluoréthyl)-1-naphtalényl]carbonyl]-N-méthylglycine; ou
la N-[[6-méthoxy-5-(tridécafluorhexyl)-1-naphtalényl]thioxométhyl]-N-méthylglycine; ou
la N-[[6-méthoxy-5-(tridécafluorhexyl)-1-naphtalényl]carbonyl]-N-méthylglycine;
ou un de ses sels thérapeutiquement acceptables, formé avec une base organique ou inorganique.

7. Procédé suivant la revendication 1 ou la revendication 3, dans lequel le composé préparé est la N-[[5-(heptafluoropropyl)-6-méthoxy-1-naphtalényl]thioxométhyl]-N-méthylglycine, ou un de ses sels thérapeutiquement acceptables, formé avec une base organique ou inorganique.

8. Procédé suivant la revendication 1 ou la revendication 2, dans lequel le composé préparé est la N-[[5-(heptafluoropropyl)-6-méthoxy-1-naphtalényl]carbonyl]-N-méthylglycine, ou un de ses sels thérapeutiquement acceptables formé avec une base organique ou inorganique.

9. Procédé suivant la revendication 1, dans lequel le composé préparé est l'ester méthylique correspondant à un composé de formule I préparé suivant l'une quelconque des revendications 6 à 8.

10. Procédé de préparation d'une composition pharmaceutique, qui consiste à associer un composé de formule I suivant la revendication 1, ou un de ses sels thérapeutiquement acceptables, à un support pharmaceutiquement acceptable.